# EUROPÄISCHE PATENTANMELDUNG

(11) **EP 4 650 144 A1**
(43) Veröffentlichungstag der Anmeldung: **19.11.2025**
(21) Anmeldenummer: 25172522.2
(22) Anmeldetag: 25.04.2025
(51) Int. Cl.: B29C 49/42, B29C 49/46, A61L 2/18, A61L 2/20, B29K 67/00, B29L 31/00, B29C 49/36, B29C 49/02, B67C 7/00, B67C 3/22

(54) **VERFAHREN UND VORRICHTUNG ZUR KONTAMINATIONSFREIEN BEHANDLUNG VON BEHÄLTERN IN EINER BEHANDLUNGSMASCHINE**

(30) Priorität: 14.05.2024 DE 102024113473
(71) Anmelder: KHS GmbH, 44143 Dortmund (DE)
(72) Erfinder: Gerhards, Martin, 44143 Dortmund (DE); Herold, Thomas, 44143 Dortmund (DE); Vetter, Elke, 44143 Dortmund (DE)
(74) Vertreter: Eisenführ Speiser

(57) **Zusammenfassung**

Die vorliegende Erfindung betrifft eine Vorrichtung und ein Verfahren zur kontaminationsfreien Behandlung von Behältern in einer Behandlungsmaschine (1), die von einem über einem Aufstellboden (13) stehenden Maschinengehäuse (11) umgeben ist, wobei die Behandlungsmaschine (1) mindestens eine erste Sektion (S1) umfasst in der eine Behandlungseinrichtung (4) für die Behälter, insbesondere ein Behandlungsrad angeordnet ist, wobei das Verfahren umfasst: Anordnen von Trennwänden (9) in der ersten Sektion (S1) und mindestens abschnittweises Anordnen der Trennwände um die Behandlungseinrichtung (4) herum zur Ausbildung eines von den Trennwänden (9) mindestens teilweise umgebenen inneren Abschnittes, Anschließen einer Luftzuführeinrichtung (7) für sterile Luft an den inneren Abschnitt und Zuführen von steriler Luft innerhalb des von den Trennwänden (9) umschlossenen inneren Abschnittes, derart dass mindestens im Bereich der Behälter (B) ein erster innerer Luftverdrängungsabschnitt (14) ausgebildet wird, wobei die Trennwände (9) mindestens abschnittweise vom Maschinengehäuse (11), bevorzugt nach innen, beabstandet angeordnet sind, so dass zwischen dem Maschinengehäuse (11) und den Trennwänden (9) äußere Absaugkanäle (12) gebildet werden, welche die Behandlungseinrichtung (4) zu einem überwiegenden Teil umgeben, wobei sich die Trennwände (9) mindestens abschnittweise entlang eines Transportpfades der Behälter (B) um die Behandlungseinrichtung (4) erstrecken und die Behälter (B) in vertikaler Richtung nach oben und unten überragen, ohne mit dem Boden (13), insbesondere Aufstellboden, abzuschließen, wobei die Absaugkanäle (12) seitlich und nach oben geschlossen sind und durch das Zuführen steriler Luft in dem ersten inneren Luftverdrängungsabschnitt (14) eine zumindest im Bereich der Behälter (B) vertikale Verdrängungsströmung (5) gebildet wird, Anschließen der äußeren Absaugkanäle (12) an eine Luftabführeinrichtung (8), Absaugen der Luft aus den äußeren Absaugkanälen (12) mittels der Luftabführeinrichtung (8), so dass eine Saugströmung in den Absaugkanälen (12) gebildet wird und Luft vom ersten inneren Luftverdrängungsabschnitt (14) in radialer Richtung zu den äußeren Absaugkanälen (12) unter den Trennwänden (9) hindurch vorbeifließt.

## Beschreibung

Die Erfindung betrifft ein Verfahren und eine Vorrichtung zur kontaminationsfreien Behandlung von Behältern in einer Behandlungsmaschine.

Bekannt ist die Herstellung von Behältern durch Blasformen aus Vorformlingen aus einem thermoplastischen Material, beispielsweise aus Vorformlingen aus PET (Polyethylenterephthalat). Dabei werden die Vorformlinge innerhalb einer Blasformmaschine als einer Art einer Vorrichtung zur Behandlung von Behältern unterschiedlichen Bearbeitungsstationen zugeführt. Typischerweise weist eine Blasformmaschine eine Heizvorrichtung zum Temperieren bzw. zum thermischen Konditionieren der Vorformlinge sowie eine Blaseinrichtung mit wenigstens einer Blasstation auf, in deren Bereich der jeweils zuvor temperaturkonditionierte Vorformling zu einem Behälter expandiert wird. Im vorliegenden Kontext stehen Streckblasmaschinen im Vordergrund, bei denen der Vorformling mittels einer Reckstrange während des Expandierens gereckt wird.

Möglichkeiten der Temperierung der Vorformlinge sind beispielsweise in der DE 23 52 926 A1 erläutert. Unter Temperierung oder thermischer Konditionierung wird dabei verstanden, dass der Vorformling auf eine für die Umformung geeignete Temperatur erwärmt und dem Vorformling ggf. ein Temperaturprofil in Längsrichtung und/oder in Umfangsrichtung aufgeprägt wird.

Die Expansion in den fertigen Behälter erfolgt beispielsweise mit Hilfe eines Druckgases, insbesondere Druckluft, als Druckmedium, das mit einem Formdruck in den zu expandierenden Vorformling eingeleitet wird. Der verfahrenstechnische Ablauf bei einer derartigen Expansion des Vorformlings wird in der DE 43 40 291 A1 erläutert. Der grundsätzliche Aufbau einer Blasstation ist in der DE 42 12 583 A1 beschrieben. Gemäß einem typischen Weiterverarbeitungsverfahren werden die durch Blasformen hergestellten Behälter einer nachfolgenden Fülleinrichtung zugeführt und hier mit dem vorgesehenen Produkt oder Füllgut gefüllt. Es ist jedoch auch möglich, Behälter aus Vorformlingen herzustellen und dabei gleichzeitig mit einem Füllgut zu befüllen, welches als hydraulisches Druckmedium zum Expandieren des Vorformlings bzw. zum Ausformen des Behälters mit einem Form- und Fülldruck zugeführt wird. Dadurch wird zeitgleich mit dem Füllen der jeweilige Vorformling in den Behälter umgeformt. In Fachkreisen sind solche Verfahren und Vorrichtungen zum gleichzeitigen Umformen und Befüllen von Vorformlingen in Behälter unter den Bezeichnungen "FormFill" oder "LiquiForm" bekannt. Die vorliegende Erfindung lässt sich vorteilhaft auf beide genannten Umformverfahren und beide Arten von Vorrichtungen zum Umformen von Vorformlingen anwenden.

Unter einem Sterilisieren (auch Sterilisation) wird vorliegend verstanden, dass z.B. unter Verwendung chemischer Sterilisierfluide eine keimtötende Behandlung erfolgt. Die vorliegende Offenbarung ist unter dem Aspekt zu sehen, dass Getränke unter aseptischen bzw. sterilen Bedingungen abgefüllt werden müssen, um die gewünschte Haltbarkeit zu erreichen. Dies setzt z.B. voraus, dass die Behälter, in die die Getränke abgefüllt werden, diese Sterilbedingungen erfüllen, also mindestens auf den füllgutberührten Oberflächen weitgehend keimfrei sind, derart dass eine Sterilisierungsbehandlung durchgeführt wurde um Mikroorganismen abzutöten. Insofern sind Keime im Kontext der vorliegenden Offenbarung als Mikroorgansimen zu verstehen. Zu diesem Zweck können fertige Behälter vor der Befüllung oder Vorformlinge vor ihrer Umformung in Behälter sterilisiert werden, wobei nachfolgend dann eine Neuverkeimung verhindert wird. So können sowohl die Vorformlinge als auch später die daraus hergestellten Behälter sterilisiert werden. Die Sterilisierung des Vorformlings hat gegenüber der Sterilisierung des daraus hergestellten Behälters den Vorteil, dass die zu sterilisierende Oberfläche viel kleiner ist, so dass die einzusetzende Menge an Sterilisierfluid geringer ausfallen kann.

Üblicherweise ist nach dem Blasformvorgang eine Spülung mit einem sterilen Spülfluid vorgesehen, um den fertigen Behälter von den Spuren des Sterilisierfluides zu befreien. Dies ist bspw. in der WO 2014/139624 A1 beschrieben. Der Blasformvorgang selbst führt bereits zu einem Spüleffekt, denn das verwendete Blasgas und das Ablassen des verwendeten Blasgases trägt zum Entfernen des Sterilisierfluides bei, soweit dieses noch vor der Umformung im Vorformling enthalten ist. Auch kann ein Sterilisierfluid, z.B. Wasserstoffperoxid, zerfallen und ist somit nicht mehr im Vorformling oder dem Behälter enthalten.

Aus der DE 10 2014 010 283 A1 ist z.B. bekannt, einen Vorformling mit einem Sterilisierfluid zu durchspülen, während der Vorformling bereits in der Umformstation aufgenommen ist. Ebenso ist es bekannt, einen Vorformling vor dem Erreichen der Umformstation einer Sterilisierung zu unterziehen (WO 2010/020530 A1), z.B. auf dem Weg zwischen der Heizvorrichtung und der Umformstation, oder z.B. vor dem Einlaufen des Vorformlings in die Heizvorrichtung einer Blasformmaschine, oder z.B. vor einem Einlauf eines Vorformlings in eine Blasformmaschine, z.B. im Bereich einer Zuführschiene für die Vorformlinge. Aus der EP 2 588 295 A1 ist auch bekannt, einen Vorformling innerhalb der Heizvorrichtung zu sterilisieren.

Eine bekannte Vorgehensweise bei der Sterilisation von Vorformlingen ist z.B., dass Wasserstoffperoxid (H₂O₂) in den Vorformling dosiert, der Vorformling zusammen mit dem dosierten Wasserstoffperoxid in der Heizvorrichtung erwärmt und im Anschluss ein fertiger Behälter geblasen wird. Dabei zerfällt der überwiegende Teil des eingesetzten Wasserstoffperoxids in Sauerstoff und Wasser. Bekannt ist aber auch, die Zudosierung von Wasserstoffperoxid zwischen der Heizvorrichtung und der Umformvorrichtung vorzusehen. Bei Entspannung auf Umgebungsdruck wird der fertige Behälter mit dem 25- bis 30-fachen des Flaschenvolumens gespült und damit etwaig verbliebenes Wasserstoffperoxid auf einen tolerierbaren Wert abgesenkt. Der Vorformling wird in entsprechender Weise auch von außen sterilisiert.

Es ist bekannt, eine Behandlung von Behältern, insbesondere einen Umformvorgang, in dem Vorformlinge zu Behältern umgeformt werden, unter sterilen Bedingungen durchzuführen. Dazu wird typischerweise ein Reinraum verwendet, innerhalb dessen die Behandlung der Behälter erfolgt, z.B. deren Herstellung durch Umformung.

Unter einem Behälter wird dabei nicht nur ein fertiger Behälter verstanden, sondern auch ein Vorformling, aus dem der fertige Behälter hergestellt wird. Für die vorliegende Anmeldung steht der Begriff "Behälter" also gleichermaßen für Vorformlinge und für daraus hergestellte fertige Behälter.

Unter einer Behandlung von Behältern wird neben der schon genannten Umformung von Vorformlingen, die auch als Behälter verstanden werden, auch z.B. das Befüllen, Verschließen, Etikettieren, Sterilisieren, Reinigen, etc. verstanden. Dieses insoweit für ein Behandeln zu verlangende Einwirken muss aber nicht mit einer Veränderung des Behälters einhergehen, sondern dieses Einwirken kann z.B. auch ein Inspizieren eines Behälters sein für eine Entscheidung, ob der Behälter auszuschleusen ist. Dieses Einwirken kann auch ein Vermessen des Behälters sein, z.B. um die erhaltenen Messwerte für eine Steuerung oder für eine Auswertung zu verwenden. Unter einem Behandeln wird aber nicht ein reiner Transport verstanden.

Es ist auch bekannt, einen Teilraum innerhalb einer Blasmaschine zu einem Reinraum zu machen, wobei sich dieser Teilraum möglichst auf diejenigen Bereiche beschränkt, in denen die Behälter geführt und behandelt werden. Einen solchen Stand der Technik zeigt z.B. die WO 2010/020529 A2, die Blasmaschinen betrifft, insbesondere solche mit Verwendung einer Reckstange, also Streckblasmaschinen, und insbesondere Maschinen rotierender Bauart. Aufgrund dieser rotierenden Bauart gibt es stationäre Reinraumwände und sich mit dem Blasrad mitbewegende Reinraumwände, sowie dazwischen angeordnete Dichtungen. In diesem Stand der Technik ist auch beschrieben, dass zur Sterilhaltung innerhalb des Reinraums ein Sterilgas vorgesehen ist und dass dieses Sterilgas unter einem Druck steht, der höher ist als ein Druck außerhalb des Reinraums. Erwähnt ist auch, dass optional zur Sterilhaltung dem Reinraum kontinuierlich ein antimikrobieller Wirkstoff zugeführt wird. Es ist dort auch erwähnt, dass sich der besagte Reinraum bis zu einer der Blasmaschine nachgelagert angeordneten Füllmaschine und bevorzugt bis in den Bereich einer Verschließeinrichtung zum Verschließen der gefüllten Behälter erstreckt. Diese WO 2010/020529 A2 beschreibt auch Möglichkeiten zur Sterilisierung der Vorformlinge.

Die zuvor genannten Verfahren und Vorrichtungen haben alle noch ihre spezifischen Nachteile und sind teilweise aufwändig und kostenintensiv. Die vorliegende Erfindung hat sich zur Aufgabe gestellt, ein effizientes und sicheres Verfahren und eine ebensolche Vorrichtung bereitzustellen, welche insbesondere eine kontaminationsfreie Behandlung von Behältern in einer Behandlungsmaschine ermöglichen.

Die vorliegende Offenbarung stellt ein Verfahren und eine Vorrichtung zur kontaminationsfreien Behandlung von Behältern in einer Behandlungsmaschine bereit.

Die Behandlungsmaschine kann von einem Maschinengehäuse umgeben sein. Unterhalb des Maschinengehäuses befindet sich ein Boden bspw. ein Hallenboden. Auf diesem Boden können bspw. ein oder mehrere Aufstellböden angeordnet sein, welche die Vorrichtungen und Einrichtungen der Behandlungsmaschine tragen. Der Hallenboden selbst kann aber auch den Aufstellboden bilden. Die Einrichtungen und Vorrichtungen werden von dem Maschinengehäuse überspannt und umschlossen. Das Maschinengehäuse schließt jedoch nicht mit dem Boden oder den Aufstellböden ab. Daher kann grundsätzlich Hallenluft von unten in das Maschinengehäuse gelangen. Das Maschinegehäuse ist also nicht in der Art eines Reinraumgehäuses auszubilden.

Gemäß der vorliegenden Erfindung kann die Behandlungsmaschine mindestens eine Sektion und vorteilhaft mehrere Sektionen aufweisen. Die Sektionen können vom Maschinengehäuse und von zusätzlichen Trennwänden umschlossen sein. Üblicherweise ist das Maschinengehäuse bzw. sind die Wände des Maschinengehäuses nicht vollständig luftdicht bzw. luftundurchlässig oder zumindest weniger luftdicht bzw. luftundurchlässig als die Trennwände.

Gemäß der vorliegenden Offenbarung werden in den Sektionen oder entlang der Sektionen durch Anbringen von jeweiligen Trennwänden innere und äußere Abschnitte gebildet, in denen durch Zuführen steriler Luft oder Abführen von Luft bestimmte vorteilhafte Strömungsverhältnisse erzeugt werden. Hierbei handelt sich, zumindest im Bereich der Behälter um bevorzugt vertikale Verdrängungsströmungen (von oben nach unten) oder um bevorzugt vertikale Saugströmungen (von unten nach oben). Die Trennwände werden gemäß der vorliegenden Offenbarung auch dazu eingesetzt, einzelne Sektionen voneinander abzugrenzen. Zur besseren Unterscheidung werden diese Trennwände in den Ansprüchen den Sektionen zugeordnet und nach diesen Sektionen benannt.

Die sterile Luft kann bspw. durch den Einsatz von Filtern in Luftleitungen bereitgestellt werden. Derartige Filter sind bekannt. Es handelt sich bspw. um sogenannte HEPA-Filter.

In den Sektionen befinden sich vorteilhaft verschiedene Vorrichtungen und Einrichtungen der Behandlungsmaschine. Dabei kann es sich um Behandlungseinrichtungen für die Behälter, wie ein Behandlungsrad (bspw. ein Blasrad, Streckblaseinrichtung), Heizvorrichtungen, Vorrichtungen zum Sterilisieren, Befüllen, Etikettieren oder Inspizieren und andere handeln, aber auch um Transfereinrichtungen.

Die Sektionen sind in bestimmter Reihenfolge über einen Transportpfad der Behälter verbunden. Mit anderen Worten können die Behälter von einer Sektion zu einer benachbarten Sektion transportiert werden. Aus diesem Grund können sich bestimmte Einrichtungen (bspw. Transporteinrichtungen) von einer Sektion in eine andere Sektion erstrecken.

Die einzelnen Sektionen werden nachfolgend in nummerisch aufsteigender Reihenfolge beschrieben. Dabei stimmt die nummerische Reihenfolge nicht mit der Reihenfolge des Durchlaufens der Behälter durch die Sektionen entlang des Transportpfades überein. Vielmehr betreffen die Nummern der Sektionen deren Reihenfolge ihrer Erwähnung innerhalb der vorliegenden Offenbarung.

In einer ersten Sektion der Behandlungsmaschine kann allgemein eine Behandlungseinrichtung für Behälter angeordnet sein. Die Behandlungseinrichtung kann eine beispielsweise eine Streckblaseinrichtung zum Umformen von Vorformlingen in fertige Behälter sein. Vorteilhaft können mindestens abschnittweise Trennwände um die Behandlungseinrichtung herum angeordnet sein. Die Trennwände, als Trennwände der ersten Sektion bezeichnet, bilden einen von den Trennwänden umgebenen bzw. umschlossenen inneren Abschnitt, als innerer Abschnitt der ersten Sektion bezeichnet. Dieser innere Abschnitt kann, muss aber nicht vollständig von den Trennwänden der ersten Sektion umschlossen sein.

An den inneren Abschnitt kann vorteilhaft eine Luftzuführeinrichtung für sterile Luft angeschlossen sein. Sterile Luft kann mittels der Luftzuführeinrichtung bspw. seitlich (oberhalb der Behälter) und/oder von oben (oberhalb der Behälter), vorteilhaft in vertikaler Richtung von oben nach unten innerhalb des von den Trennwänden umschlossenen inneren Abschnittes zugeführt werden bzw. strömen. Dadurch bildet sich im inneren Abschnitt ein erster innerer Luftverdrängungsabschnitt aus. In dem inneren Luftverdrängungsabschnitt herrscht eine Luftverdrängungsströmung. Die Behandlungseinrichtung befindet sich vorteilhaft innerhalb dieses Luftverdrängungsabschnitts bzw. wird von diesem aufgenommen. Der innere Luftverdrängungsabschnitt sollte mindestens im Bereich der Behälter erzeugt werden.

Vorteilhaft sind die Trennwände in ihrer Höhe so dimensioniert, dass sie die Behälter in vertikaler Richtung nach oben und unten (entlang des Transportpfades durch die Behandlungseinrichtung) überragen. Nach oben können die Trennwände mit dem Maschinengehäuse abschließen, also nach oben geschlossen sein.

Unterhalb der Trennwände, also zwischen dem unteren Rand der Trennwände und einem Boden (Aufstellboden, Bodenbleche etc.), bleibt mindestens ein Spalt frei. Die Trennwände schließen also nicht mit dem Boden oder den Böden ab, sondern bleiben nach unten vom Boden beabstandet.

Vorzugsweise befinden sich die Trennwände innerhalb des vom Maschinengehäuse umhausten Bereiches. Nach außen, also zum Maschinengehäuse hin, können die Trennwände vorteilhaft mindestens abschnittweise vom Maschinengehäuse beabstandet sein. Dadurch werden Räume bzw. Kanäle zwischen einer oder mehreren Trennwänden und den Wänden des Maschinengehäuses gebildet, die als äußere Absaugkanäle bezeichnet werden, vorliegend als äußere Absaugkanäle der ersten Sektion.

Vorteilhaft sind diese äußeren Absaugkanäle (bspw. durch die Trennwände) seitlich und nach oben geschlossen. Diese äußeren Absaugkanäle sind an eine Absaugeinrichtung angeschlossen. Die Absaugeinrichtung kann seitlich und/oder von oben an die Absaugkanäle angeschlossen sein. In den Absaugkanälen der ersten Sektion wird mittels einer Absaugeinrichtung seitlich und/oder von oben Luft abgesaugt. Dadurch ergibt sich eine Saugströmung innerhalb der Absaugkanäle, die bevorzugt vertikal sein kann.

Die Trennwände und/oder die Absaugkanäle können die Behandlungseinrichtung zu einem überwiegenden Teil (vorteilhaft von mehr als 180° Umfangswinkel in Umfangsrichtung der Behandlungseinrichtung) umgeben.

In einer Ausgestaltung kann die Behandlungseinrichtung an mindestens drei Seiten von Absaugkanälen umgeben sein. Dabei erstrecken sich die Trennwände mindestens abschnittweise entlang eines Transportpfades der Behälter um die Behandlungseinrichtung herum.

Insgesamt wird also vorteilhaft durch das Zuführen steriler Luft in dem ersten inneren Luftverdrängungsabschnitt ein zumindest im Bereich der Behälter vertikaler

Verdrängungsluftstrom (Luftverdrängungsströmung) von oben nach unten gebildet.

Durch das Absaugen der Luft aus den äußeren Absaugkanälen der ersten Sektion wird in der ersten Sektion ein bevorzugt vertikaler Saugluftstrom (Saugströmung) von Luft in den Absaugkanälen von unten nach oben gebildet. Vorteilhaft führt das dazu, dass Luft vom ersten inneren Luftverdrängungsabschnitt (bspw. in radialer Richtung) zu dem oder den äußeren Absaugkanälen der ersten Sektion unter den Trennwänden der ersten Sektion hindurch vorbeifließt.

Hierdurch wird vorteilhaft verhindert, dass die Behälter beim Durchlaufen der Behandlungseinrichtung in der ersten Sektion mit kontaminierter Luft in Berührung kommen können, da die Behälter nur mit steriler Luft aus den Luftzuführeinrichtungen in Berührung kommen, wobei die Luft anschließend durch die Absaugkanäle der ersten Sektion radial nach außen abgeführt wird. Die Luft strömt somit zumindest im Bereich der Behälter von oben nach unten ein, strömt unterhalb der Trennwände radial nach außen und wird von der Behandlungseinrichtung aus betrachtet radial außen nach oben abgesaugt und abgeführt. Es entsteht eine vorteilhafte Luftbewegung steriler Luft zunächst von oben auf die Behälter zu und dann weg von den Behältern nach unten und radial nach außen.

Die Richtungen der Luftströmungen werden im Rahmen der vorliegenden Offenbarung idealisiert als vertikal von oben nach unten oder unten nach oben beschrieben. Natürlich werden durch die Bewegung der Einrichtungen und Vorrichtungen innerhalb der Sektionen in manchen Abschnitten Verwirbelungen erzeugt. Die Stärke der Luftströmungen ist vorteilhaft auf diese Effekte abgestimmt. Dies gilt in gleicher Weise für die Angaben, dass ein Strömen nach außen bzw. radial nach außen erfolgt. Auch dies ist eine Idealisierung, die aber die generelle Strömungscharakteristik zutreffend beschreibt.

Die Behandlungsmaschine kann eine zweite Sektion umfassen, die eine erste Transfereinrichtung, insbesondere ein Zuführrad für Behälter, zumindest teilweise aufnimmt. Ein Teil der ersten Transfereinrichtung kann aus der zweiten Sektion herausragen und sich in die erste Sektion erstrecken, was zum Transport von Behältern von der zweiten in die erste Sektion vorteilhaft sein kann.

In der zweiten Sektion und/oder entlang der zweiten Sektion können Trennwände (nachfolgend als Trennwände der zweiten Sektion bezeichnet) angeordnet sein, die sich mindestens abschnittweise um die Transfereinrichtung herum erstrecken. Dadurch wird ein von den Trennwänden der zweiten Sektion mindestens teilweise umgebener innerer Abschnitt gebildet, nachfolgend innerer Abschnitt der zweiten Sektion. Eine Luftzuführeinrichtung für sterile Luft kann seitlich und/oder von oben an diesen inneren Abschnitt der zweiten Sektion angeschlossen werden. Mittels der Luftzuführeinrichtung kann sterile Luft innerhalb des von den Trennwänden der zweiten Sektion umschlossenen inneren Abschnittes der zweiten Sektion zugeführt werden, und zwar so, dass dort ein zweiter innerer Luftverdrängungsabschnitt ausgebildet wird, der mindestens teilweise die erste Transfereinrichtung umgibt.

Ein Teil der Trennwände der zweiten Sektion kann mindestens abschnittweise vom Maschinengehäuse, vorzugsweise nach innen, beabstandet angeordnet sein, so dass zwischen dem Maschinengehäuse und den Trennwänden der zweiten Sektion mindestens ein äußerer Absaugkanal der zweiten Sektion gebildet wird, wobei der Absaugkanal der zweiten Sektion seitlich und nach oben geschlossen ist. Das Maschinengehäuse befindet sich dabei vorteilhaft radial außen bzw. die Trennwände befinden sich vorteilhaft innerhalb des vom Maschinengehäuse umhausten Bereichs.

Die Trennwände der zweiten Sektion können vorteilhaft die Behälter in vertikaler Richtung nach oben und unten überragen, ohne mit dem Boden, insbesondere Aufstellboden, abzuschließen. Nach oben können die Trennwände der zweiten Sektion mit dem Maschinengehäuse abschließen, also nach oben geschlossen sein.

Durch das Zuführen steriler Luft in den zweiten inneren Luftverdrängungsabschnitt wird eine zumindest im Bereich der Behälter bevorzugt vertikale Verdrängungsströmung erzeugt.

Eine Luftabführeinrichtung kann dann seitlich und/oder von oben an den äußeren Absaugkanal der zweiten Sektion angeschlossen werden, durch die Luft aus diesem äußeren Absaugkanal abgesaugt wird, so dass eine, bevorzugt vertikale von unten nach oben gerichtete, Saugströmung in dem Absaugkanal gebildet wird und Luft vom ersten inneren Luftverdrängungsabschnitt in radialer Richtung zu dem äußeren Absaugkanal der zweiten Sektion unter den Trennwänden der zweiten Sektion hindurch vorbeifließt.

Zwischen der ersten Sektion und der zweiten Sektion sind vorteilhaft ebenfalls Trennwände angeordnet, nachfolgend ebenfalls Trennwände der zweiten Sektion genannt, die nicht mit dem Boden abschließen und zusätzlich Durchbrechungen aufweisen, durch welche die Behälter von der zweiten Sektion zur ersten Sektion transportiert werden können.

In dem zweiten inneren Luftverdrängungsabschnitt der zweiten Sektion können noch weitere Transfereinrichtungen mindestens teilweise angeordnet sein, welche eingerichtet sind, um Behälter zu anderen Sektionen hin oder von anderen Sektionen weg zu transportieren.

Die Behandlungsmaschine kann eine dritte Sektion umfassen, die eine zweite Transfereinrichtung, insbesondere ein Abführrad für Behälter, zumindest teilweise aufnimmt. Ein Teil der zweiten Transfereinrichtung kann aus der dritten Sektion herausragen und sich in die erste Sektion erstrecken, was zum Transport von Behältern von der ersten in die dritte Sektion vorteilhaft sein kann.

In der dritten Sektion können Trennwände (Trennwände der dritten Sektion) angeordnet sein, die sich mindestens abschnittweise um die Transfereinrichtung herum erstrecken. Dadurch wird ein von den Trennwänden der dritten Sektion mindestens teilweise umgebener innerer Abschnitt der dritten Sektion gebildet. Eine Luftzuführeinrichtung für sterile Luft kann seitlich und/oder von oben an diesen inneren Abschnitt angeschlossen werden. Mittels der Luftzuführeinrichtung kann sterile Luft bevorzugt von oben nach unten innerhalb des von den Trennwänden der dritten Sektion umschlossenen inneren Abschnittes der dritten Sektion zugeführt werden, so dass dort ein dritter innerer Luftverdrängungsabschnitt ausgebildet wird, der mindestens teilweise die erste Transfereinrichtung umgibt.

Ein Teil der Trennwände der dritten Sektion kann mindestens abschnittweise vom Maschinengehäuse, vorzugsweise nach innen, beabstandet angeordnet sein, so dass zwischen dem Maschinengehäuse und den Trennwänden der dritten Sektion mindestens ein äußerer Absaugkanal der dritten Sektion gebildet wird, wobei dieser Absaugkanal vorteilhaft seitlich und nach oben geschlossen ist.

Die Trennwände der dritten Sektion können vorteilhaft die Behälter in vertikaler Richtung nach oben und unten überragen, ohne mit dem Boden, insbesondere Aufstellboden, abzuschließen. Nach oben können die Trennwände mit dem Maschinengehäuse abschließen, also nach oben geschlossen sein.

Durch das Zuführen steriler Luft in den dritten inneren Luftverdrängungsabschnitt wird zumindest im Bereich der Behälter eine (weitgehend) vertikale Verdrängungsströmung erzeugt.

Eine Luftabführeinrichtung kann dann seitlich und/oder von oben an den äußeren Absaugkanal der dritten Sektion angeschlossen werden, durch die Luft aus diesem äußeren Absaugkanal abgesaugt wird, so dass eine, bevorzugt vertikale von unten nach oben gerichtete, Saugströmung in dem Absaugkanal gebildet wird und Luft vom dritten inneren Luftverdrängungsabschnitt in radialer Richtung zu dem äußeren Absaugkanal der dritten Sektion unter den Trennwänden der dritten Sektion hindurch vorbeifließt.

Zwischen der ersten Sektion und der dritten Sektion sind vorteilhaft ebenfalls Trennwände (der dritten Sektion) angeordnet, die nicht mit dem Boden abschließen und zusätzlich Durchbrechungen aufweisen, durch welche die Behälter von der ersten Sektion zur dritten Sektion transportiert werden können.

In dem dritten inneren Luftverdrängungsabschnitt der dritten Sektion können noch weitere Transfereinrichtungen mindestens teilweise angeordnet sein, welche eingerichtet sind, um Behälter zu oder von anderen Sektionen zu transportieren.

Die Behandlungsmaschine kann eine vierte Sektion umfassen, in der eine Heizvorrichtung angeordnet ist. Zwischen der vierten Sektion und der zweiten Sektion können Trennwände (nachfolgend: Trennwände der vierten Sektion) angeordnet sein, wobei diese Trennwände der vierten Sektion mit dem Boden abschließen und Durchbrechungen aufweisen, durch welche die Behälter von der vierten Sektion zur zweiten Sektion transportiert werden können und umgekehrt. Eine Transporteinrichtung, vorzugsweise eine Transporteinrichtung der Heizvorrichtung, kann sich dazu von der vierten Sektion in die zweite Sektion erstrecken.

Eine Luftabführeinrichtung kann von oben an die vierte Sektion angeschlossen werden, um mittels dieser Luft aus der vierten Sektion abzusaugen, so dass in der vierten Sektion eine vertikale von unten nach oben gerichtet Saugströmung eingerichtet wird, welche die Heizvorrichtung durchströmt.

Die Luft kann ebenfalls von der zweiten Sektion (Verdrängungsströmung) zur vierten Sektion (Saugströmung) durch die Durchbrechungen der Trennwände der vierten Sektion hindurchfließen.

Unterhalb der Heizvorrichtung können Luftfilter (bspw. HEPA-Filter) angeordnet sein, welche die durch den Boden oder seitlich angesaugte Luft filtern, bevor diese durch die Heizvorrichtung strömt. Die Luft dient hier auch zum Kühlen.

Die Behandlungsmaschine kann eine fünfte Sektion umfassen, in der weitere Transfereinrichtungen angeordnet sind. Zunächst können hier Trennwände (nachfolgend: Trennwände der vierten Sektion) zwischen der fünften Sektion und der zweiten Sektion angeordnet werden, wobei diese Trennwände mit dem Boden abschließen und Durchbrechungen (für eine Transporteinrichtung) aufweisen, durch welche die Behälter von der fünften Sektion zur zweiten Sektion transportiert werden können. Hier befindet sich eine durchgehende Bodenplatte unterhalb der Vorformlinge bzw. unterhalb der Trennwände, so dass keine Hallenluft eingesaugt werden kann. Diese Bodenplatte kann die Form eines zusätzlich eingezogenen Zwischenbodens haben.

Innerhalb der fünften Sektion können weitere Trennwände der fünften Sektion angeordnet sein, die sich um die weiteren Transfereinrichtungen herum erstrecken, wobei diese Trennwände der fünften Sektion mindestens abschnittweise vom Maschinengehäuse, vorzugsweise nach innen, beabstandet angeordnet sein können. Vorteilhaft bilden die Trennwände der fünften Sektion einen oder mehrere innere Abschnitte der fünften Sektion.

Auch in der fünften Sektion können die Trennwände der fünften Sektion die Behälter auf ihrem Transportpfad in vertikaler Richtung nach oben und unten überragen, wobei diese Trennwände hier mit dem Boden, insbesondere Zwischenboden, abschließen. Nach oben können die Trennwände mit dem Maschinengehäuse abschließen, also nach oben geschlossen sein.

Die inneren Abschnitte der fünften Sektion können von oben an eine Luftabführeinrichtung angeschlossen werden. Durch diese kann Luft aus diesen inneren Abschnitten mittels der Luftabführeinrichtung abgesaugt werden, so dass innere Saugströmungsabschnitte gebildet werden, in denen eine vertikale von unten nach oben gerichtete Saugströmung herrscht.

Auch in der fünften Sektion befinden sich die Trennwände vorteilhaft innerhalb eines von dem Maschinengehäuse umhausten Bereiches. Die Trennwände können hier eine geschlossene Kammer bilden, die im nachfolgenden als sechste Sektion beschrieben ist. Diese sechste Sektion kann eine Sterilisationseinrichtung (Sterilisationskammer) sein.

Unterhalb der fünften und sechsten Sektion befindet sich eine feste, undurchlässige Bodenplatte, bzw. ein zusätzlicher Zwischenboden, mit dem die Trennwände nach unten abschließen.

Die Behandlungsmaschine kann eine sechste Sektion umfassen, in welcher eine Beaufschlagungseinrichtung für Sterilisierfluid (oder auch Sterilisiervorrichtung für Behälter) angeordnet ist, mit der die Behälter mit dem Sterilisierfluid beaufschlagt werden.

Gemäß einem Aspekt kann die sechste Sektion innerhalb der fünften Sektion durch Anordnen von Trennwänden (nachfolgend: Trennwände der sechsten Sektion, Sterilisationskammer) um die Beaufschlagungseinrichtung für Sterilisierfluid herum gebildet werden. Auch hier können die Trennwände der sechsten Sektion die Behälter nach oben und unten überragen. Diese Trennwände schließen hier mit dem Boden, bzw. luftundurchlässigen Zwischenboden, ab. Nach oben können die Trennwände die sechste Sektion mit dem Maschinengehäuse abschließen, also nach oben geschlossen sein. Eine Luftzuführeinrichtung kann von oben an die sechste Sektion angeschlossen werden, derart dass mittels der Luftzuführeinrichtung in der sechsten Sektion eine vertikale von oben nach unten gerichtete Verdrängungsströmung herrscht.

In der Behandlungsmaschine kann eine siebte Sektion vorgesehen sein, die bspw. zwei weitere Transfereinrichtung teilweise umgibt, wobei die siebte Sektion stromabwärts (bezüglich des Transportpfades der Behälter) der dritten Sektion angeordnet ist. Mindestens eine Transfereinrichtung kann sich von der dritten Sektion in die siebte Sektion erstrecken bzw. teils in der dritten und teils in der siebten Sektion angeordnet sein.

Weitere Trennwände (Nachfolgend: Trennwände der siebten Sektion) können vorteilhaft zwischen der siebten Sektion und der dritten Sektion angeordnet werden, wobei diese Trennwände der siebten Sektion mit dem Boden abschließen und zusätzlich Durchbrechungen (für die Transporteinrichtung) aufweisen, durch welche die Behälter mit der Transfereinrichtung von der dritten Sektion zur siebten Sektion transportiert werden können. Die siebte Sektion kann vorteilhaft zwischen einer Behälterbehandlungseinheit, wie einer Streckblaseinrichtung, und einer Fülleinheit (Füller) liegen. In der Fülleinheit herrschen besonders hohe Anforderungen an die Sterilität. Daher schließen die Trennwände in der siebten Sektion fest mit dem Boden ab und der Boden ist undurchlässig für Hallenluft bzw. Luft von außen. Das gilt auch für die weiteren umgebenden Wände der siebten Sektion.

In einer Ausgestaltung kann eine Luftabführeinrichtung an die siebte Sektion von oben oder von unten angeschlossen sein. Somit kann Luft aus der siebten Sektion mittels der Luftabführeinrichtung abgesaugt werden, so dass in der siebten Sektion eine Saugströmung ausgebildet wird, wobei die Luft ebenfalls von der dritten Sektion zur siebten Sektion durch die Durchbrechungen hindurchfließt. Die siebte Sektion kann als Schleuse (Luftschleuse) im Hinblick auf die dritte Sektion verstanden werden.

In der Behandlungsmaschine kann eine achte Sektion vorgesehen sein, in der mindestens eine Zuführeinrichtung für Behälter, insbesondere Vorformlinge angeordnet ist.

Die achte Sektion kann stromaufwärts (bezüglich der Transportrichtung der Behälter) der fünften Sektion angeordnet sein. Trennwände (nachfolgend Trennwände der achten Sektion) können vorteilhaft zwischen der achten Sektion und der fünften Sektion angeordnet werden, wobei diese Trennwände der achten Sektion zusätzlich Durchbrechungen aufweisen können, durch welche die Behälter von der achten Sektion zur fünften Sektion transportiert werden können.

Eine Luftzuführeinrichtung kann an die achte Sektion von oben, unten oder auch seitlich angeschlossen bzw. eingerichtet werden, um Luft in die achte Sektion mittels der Luftzuführeinrichtung zuzuführen, so dass in der achten Sektion eine Verdrängungsströmung eingerichtet wird. Die achte Sektion ist dabei so ausgebildet, dass sich dort ein Luftpolster (Überdruck, Verdrängungsströmung) bildet und die Luft sowohl in Richtung der fünften Sektion (also in Förderrichtung der Vorformlinge) als auch entgegen der Förderrichtung der Vorformlinge strömt. So wird in der achten Sektion ein Luftpolster erzeugt, welches das Einströmen von Hallenluft verhindert.

Die vorliegende Offenbarung stellt auch eine Vorrichtung zur kontaminationsfreien Behandlung von Behältern bereit, die auch als Behandlungsmaschine bezeichnet wird. Die Vorrichtung bzw. Behandlungsmaschine kann von einem über einem oder mehreren Aufstellböden stehenden Maschinengehäuse umgeben sein.

Die Behandlungsmaschine (z.B. Streckblasmaschine) kann mindestens eine erste Sektion umfassen, in der eine Behandlungseinrichtung, insbesondere ein Behandlungsrad (z.B. ein Streckblasrad), angeordnet ist.

Die Vorrichtung umfasst Trennwände, die mindestens abschnittweise um die Behandlungseinrichtung herum angeordnet sind, um einen von diesen Trennwänden der ersten Sektion mindestens teilweise umgebenen inneren Abschnitt der ersten Sektion zu bilden.

Die Vorrichtung kann eine Luftzuführeinrichtung für sterile Luft umfassen, die seitlich und/oder von oben an den inneren Abschnitt der ersten Sektion angeschlossen und eingerichtet ist, sterile Luft innerhalb des von den Trennwänden der ersten Sektion umschlossenen inneren Abschnittes zuzuführen. Durch das Zuführen von steriler Luft in den inneren Abschnitt der ersten Sektion wird ein erster innerer Luftverdrängungsabschnitt ausgebildet, der die Behandlungseinrichtung umgibt.

Die Trennwände der ersten Sektion können mindestens abschnittweise vom Maschinengehäuse, vorzugsweise nach innen, beabstandet angeordnet sein, so dass zwischen dem Maschinengehäuse und den Trennwänden der ersten Sektion äußere Absaugkanäle der ersten Sektion gebildet werden, welche die Behandlungseinrichtung zu einem überwiegenden Teil (bspw. von mehr als 180° Umfangswinkel in Umfangsrichtung) umgeben. Die Trennwände der ersten Sektion können sich mindestens abschnittweise entlang eines Transportpfades der Behälter um die Behandlungseinrichtung erstrecken und die Behälter in vertikaler Richtung nach oben und unten überragen, ohne mit dem Boden, insbesondere Aufstellboden, abzuschließen. Vorzugsweise befinden sich die Trennwände innerhalb eines vom Maschinengehäuse umhausten Bereiches.

Die Absaugkanäle der ersten Sektion sind vorteilhaft seitlich und nach oben geschlossen. Durch das Zuführen steriler Luft in dem ersten inneren Luftverdrängungsabschnitt wird zumindest im Bereich der Behälter eine vertikale Verdrängungsströmung gebildet.

Die Vorrichtung kann eine Luftabführeinrichtung umfassen, die an die äußeren Absaugkanäle der ersten Sektion seitlich und/oder von oben angeschlossen ist und eingerichtet ist, um Luft aus diesen äußeren Absaugkanälen abzusaugen, so dass eine, bevorzugt von unten nach oben gerichtete, Saugströmung in den Absaugkanälen der ersten Sektion gebildet wird. Dadurch kann Luft vom ersten inneren Luftverdrängungsabschnitt in radialer Richtung zu den äußeren Absaugkanälen der ersten Sektion unter den Trennwänden der ersten Sektion hindurch vorbeifließen.

Wie zuvor dargestellt herrschen in den Sektionen jeweils abschnittweise Saugströmungen und Verdrängungsströmungen, die je nach Sektion oder Abschnitt unterschiedliche Größen/Stärken haben können.

In den ersten, zweiten und dritten inneren Verdrängungsabschnitten der ersten bis dritten Sektion können individuell einstellbare Verdrängungsströmungen herrschen. In den Absaugkanälen können ebenfalls individuell einstellbare Größen der Saugströmungen herrschen. Auch die Saugströmungen in der vierten Sektion und der siebten Sektion können individuell eingerichtet sein.

In der fünften Sektion kann im äußeren Saugströmungsabschnitt (also außerhalb der Trennwände) ebenfalls eine Saugströmung mit einer individuellen Größe eingerichtet sein. In der sechsten Sektion, die einen Unterabschnitt der fünften Sektion darstellen kann, herrscht ebenfalls eine Verdrängungsströmung mit individueller Größe, wobei die Größe der Verdrängungsströmung in der sechsten Sektion kleiner sein kann als die Größe der Verdrängungsströmung in der zweiten Sektion. In den inneren Saugströmungsabschnitten der fünften Sektion herrscht ebenfalls eine Saugströmung mit individueller Größe, die wiederum größer sein kann als die Saugströmung außerhalb der Trennwände. Eine Verdrängungsströmung individueller Größe kann auch in der achten Sektion herrschen, also im Bereich der Zuführung kurz vor dem Eintritt in die fünfte Sektion. Die genannten Größen der Strömungen sorgen für Vorzugsströmungsrichtungen der Luft, indem definierte Druckgefälle ausgebildet werden. Innen im Bereich der Behandlungseinrichtung (Behandlungsrad) soll der Druck am größten sein und nach außen hin soll er abnehmen. Damit bewegt sich die sterile Luft von innen nach außen, in Richtung weg von der Behandlungseinrichtung. Dadurch wird der Bereich in der ersten, zweiten und dritten Sektion möglichst frei von Mikroorganismen gehalten.

Insgesamt wird durch die vorliegende Offenbarung ein Verfahren und eine Vorrichtung bereitgestellt, bei denen innere Verdrängungsabschnitte von äußeren Absaugabschnitten umgeben sind. Ferner sind Sektionen, wie die erste, zweite, dritte und sechste Sektion, die eine innere Verdrängungsströmung aufweisen, von Sektionen, wie der vierten, siebten und fünften Sektion, umgeben, die eine Saugströmung aufweisen. Diese Anordnung und diese Ausbildung der jeweiligen Strömungsrichtungen ermöglicht einen hochgradig kontaminationsfreien Transport und Behandlung der Behälter B innerhalb der Behandlungsmaschine.

Die Trennwände können vorteilhaft luftdicht und starr sein. Sie können aus Kunststoff oder Metall oder aus einer Kombinationen dieser Materialien bestehen. Besonders vorteilhaft können die Trennwände auch - wenigstens in bestimmten Sektionen oder Bereichen von Sektionen - aus transparentem Kunststoff bestehen. Das ermöglicht zusätzlich einen besseren Überblick, da die Trennwände zumindest abschnittweise (oder insgesamt) durchsichtig sind. Insbesondere können transparente Kunststoff-Trennwände im Bereich der ersten Sektion, der zweiten Sektion und dritten Sektion eingesetzt werden. Dort können die Trennwände aus transparentem Kunststoffvorteilhaft zwischen den Sektionen eingesetzt werden.

Die vorliegende Offenbarung stellt in einer möglichen Ausgestaltung entlang der Sektionen einen hochgradig kontaminationsfreien Transportpfad bereit. Entlang dieses kontaminationsfreien Transportpfades erfolgt der Transport der Vorformlinge und Behälter stromabwärts in der folgenden Weise. Im Rahmen einer Zuführung, z.B. ausgebildet als Zuführschiene oder als Luftförderer, durchlaufen die Vorformlinge zunächst die achte Sektion. Von dort gelangen sie zur fünften Sektion, durch die sie mittels Transfereinrichtungen (Transferräder) gefördert werden. Die sechste Sektion ist innerhalb der fünften Sektion angeordnet (und dort durch Trennwände von der fünften Sektion abgetrennt) und wird von den Behältern im Bereich der Transfereinrichtung durchlaufen. Von der letzten Transfereinrichtung der fünften Sektion gelangen die Behälter zur zweiten Sektion und dort zu einer Transfereinrichtung der zweiten Sektion. In der zweiten Sektion werden die Behälter an eine Transporteinrichtung übergeben, die sich von der zweiten Sektion in die vierte Sektion erstreckt und die Behälter in der vierten Sektion durch die Heizvorrichtung transportiert. Von der vierten Sektion und der dort angeordneten Heizvorrichtung gelangen die Behälter wieder zur zweiten Sektion und werden dort an die erste Transfereinrichtung (Transferrad, Zuführrad) übergeben. Entlang (mittels) der ersten Transfereinrichtung gelangen die Behälter zur ersten Sektion und dort werden sie von der ersten Transfereinrichtung an die Behandlungseinrichtung (bspw. Umformeinrichtung) übergeben. Nach dem Durchlaufen der Behandlungseinrichtung werden die dann fertig geformten Behälter noch innerhalb der ersten Sektion an die zweite Transfereinrichtung (Transferrad, Abführrad) übergeben und gelangen entlang der zweiten Transfereinrichtung in die dritte Sektion. In der dritten Sektion werden die Behälter von der zweiten Transfereinrichtung an eine weitere Transfereinrichtung (Transferrad) übergeben. Entlang dieser Transfereinrichtung gelangen die Behälter in die siebte Sektion. In der siebten Sektion erfolgt die Übergabe von einer weiteren Transfereinrichtung an die nächste Transfereinrichtung (Transferrad). Entlang dieser weiteren Transfereinrichtung gelangen die Behälter in die neunte Sektion. Hier kann die Behandlung beendet sein, oder es können sich weiter Behandlungen der Behälter anschließen.

Wenn die Sterilisation der Vorformlinge erst im Anschluss an die vierte Sektion stattfinden soll, dann befinden sich die fünfte und sechste Sektion zwischen der zweiten Sektion und ersten Sektion.

Nachfolgend wird die Offenbarung anhand von bevorzugten Ausführungsbeispielen und anhand der beiliegenden Figuren näher erläutert. Die Zeichnungen sind nicht unbedingt maßstabsgetreu. In den Figuren sind gleiche oder im Wesentlichen funktionsgleiche bzw. -ähnliche Elemente üblicherweise mit den gleichen Bezugszeichen bezeichnet. Es zeigen:
- Fig. 1: eine vereinfachte schematische Draufsicht einer Vorrichtung zum Behandeln von Behältern unter Sterilbedingungen am Beispiel einer Behandlungsvorrichtung zur Herstellung von fertigen Behältern aus Vorformlingen,
- Fig. 2: eine vereinfachte schematische seitliche Ansicht der Vorrichtung zum Behandeln von Behältern unter Sterilbedingungen aus Fig.1, und
- Fig. 3: eine weitere vereinfachte schematische seitliche Ansicht der Vorrichtung zum Behandeln von Behältern unter Sterilbedingungen aus Fig.1.

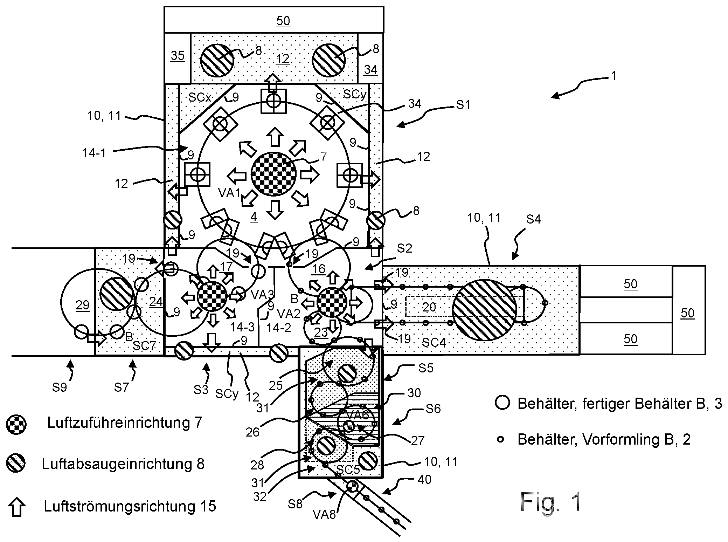

Fig. 1 ist eine vereinfachte schematische Draufsicht einer Vorrichtung 1 (Behandlungsmaschine) zum Behandeln von Behältern unter Sterilbedingungen am Beispiel einer Behandlungsmaschine 1 zur Herstellung von fertigen Behältern 3 aus Vorformlingen 2.

Die Behandlungsmaschine 1 ist von einem Maschinengehäuse umgeben und umfasst mehrere Sektionen. Beispielhaft sind hier die Sektionen S1, S2, S3, S4, S5, S6, S7 und S9 dargestellt. In anderen Ausführungen können es weniger oder auch mehr Sektionen sein. Die Sektionen werden teilweise vom Maschinengehäuse und teilweise von Trennwänden 9 räumlich begrenzt, wie später noch im Einzelnen erläutert wird. Vorteilhaft sind die Trennwände innerhalb des vom Maschinengehäuses umhausten Bereiches angeordnet bzw. das Maschinengehäuse 11 befindet sich außerhalb der Trennwände.

Entlang eines kontaminationsfreien Transportpfades erfolgt der Transport der Vorformlinge B,2 und Behälter B,3 in der folgenden Weise stromabwärts. Im Rahmen einer Zuführung 40, z.B. ausgebildet als Zuführschiene oder als Luftförderer, durchlaufen die Vorformlinge B,2 zunächst die achte Sektion S8. Von dort gelangen sie zur fünften Sektion S5, durch die sie mittels der Transfereinrichtungen 28, 27, 26 und 25 (Transferräder) gefördert werden. Die sechste Sektion S6 ist innerhalb der fünften Sektion S5 angeordnet und wird von den Behältern B,2 im Bereich der Transfereinrichtung 27 durchlaufen. Von der Transfereinrichtung 25 der fünften Sektion S5 gelangen die Behälter B,2 zur zweiten Sektion S2 und dort zur Transfereinrichtung 23. In der zweiten Sektion S2 werden die Behälter B,2 an eine Transporteinrichtung übergeben, die sich von der zweiten Sektion S2 in die vierte Sektion S4 erstreckt und die Behälter B,2 in der vierten Sektion S4 durch die Heizvorrichtung 20 transportiert. Von der vierten Sektion S4 und der dort angeordneten Heizvorrichtung 20 gelangen die Behälter B,2 wieder zur zweiten Sektion S2 und werden dort an die erste Transfereinrichtung 16 (Transferrad, Zuführrad) übergeben. Auf der Transfereinrichtung 16 gelangen die Behälter B,2 zur ersten Sektion S1 und dort werden sie von der Transfereinrichtung 16 an die Behandlungseinrichtung 4 (bspw. Umformeinrichtung) übergeben. Nach dem Durchlaufen der Behandlungseinrichtung 4 werden die Behälter B,3 noch innerhalb der ersten Sektion S1 an die zweite Transfereinrichtung 17 (Transferrad, Abführrad) übergeben und gelangen entlang der zweiten Transfereinrichtung 17 in die dritte Sektion S3. In der dritten Sektion S3 werden die Behälter B,3 von der zweiten Transfereinrichtung 17 an eine weitere Transfereinrichtung 24 (Transferrad) übergeben. Entlang dieser Transfereinrichtung 24 gelangen die Behälter B,3 in die siebte Sektion S7. In der siebten Sektion S7 erfolgt die Übergabe von der Transfereinrichtung 24 an die Transfereinrichtung 29 (Transferrad). Entlang dieser Transfereinrichtung 29 gelangen die Behälter B,3 in die neunte Sektion S9. Hier können sich weiter Behandlungen der Behälter B,3 anschließen.

Zumindest die Sektionen S1 bis S8 sind gemäß der vorliegenden Offenbarung individuell so eingerichtet und konfiguriert, dass in einer oder mehreren der jeweiligen Sektionen S1 bis S8 bestimmte vertikale Luft-Strömungsverhältnisse herrschen. Vorteilhaft werden (Luft-)Verdrängungsströmungen 5 und (Luft-)Saugströmungen 6 unterschieden. Dabei ist zu beachten, dass die Verdrängungsströmungen 5 und Saugströmungen 6 überall unterschiedliche, individuell einstellbare Größen haben.

In wenigstens einer oder mehreren der Sektionen herrscht eine Verdrängungsströmung 5 und in wenigstens einer oder mehreren der Sektionen herrscht eine Saugströmung 6.

Ungeachtet des zuvor beschriebenen Transportpfades, dem die Behälter B durch die Behandlungsmaschine 1 von Sektion zu Sektion folgen, werden die Sektion S1 bis S8 nachfolgend nochmals in numerischer Reihenfolge beschrieben.

In der ersten Sektion S1 erfolgt eine Behandlung der Behälter B. Im vorliegenden Fall handelt es sich dabei um eine Umformung von Vorformlingen 2 aus thermoplastischem Material zu fertigen Behältern 3, bevorzugt und verallgemeinerungsfähig unter Verwendung einer Reckstange. Entsprechend ist in der ersten Sektion S1 eine Behandlungseinrichtung (bspw. Behandlungsrad, Umformeinrichtung, Umformrad, Blasrad, Blaseinrichtung, Streckumformung, Streckblaseinrichtung) 4 vorgesehen. Der Aufbau und die Arbeitsweise einer solchen Behandlungseinrichtung 4 ist grundsätzlich bekannt.

Oberhalb der ersten Sektion S1 ist eine Luftzuführeinrichtung 7 für sterile Luft 18 angeordnet. Mittels der Luftzuführeinrichtung 7 wird sterile Luft 18 von oben (oder auch seitlich) in die erste Sektion S1 eingebracht. Die Luftzuführeinrichtung 7 sollte sich dabei vorteilhaft mindestens oberhalb der Behandlungseinrichtung 4 und ebenfalls vorteilhaft mindestens oberhalb der Behälter B auf ihrem Transportpfad durch die Behandlungseinrichtung befinden.

In der ersten Sektion S1 sind Trennwände 9 vorgesehen. Diese Trennwände 9 der ersten Sektion umgeben die Behandlungseinrichtung 4 zu einem überwiegenden Teil. Dadurch wird ein innerer Abschnitt gebildet. Die Luftzuführeinrichtung 7 bringt die sterile Luft 18 in diesen inneren Abschnitt der ersten Sektion ein. Dadurch wird eine Verdrängungsströmung 5 in dem inneren Abschnitt erzeugt, weshalb dieser Abschnitt als erster innerer Luftverdrängungsabschnitt 14-1 in der ersten Sektion S1 bezeichnet wird. In dem inneren Luftverdrängungsabschnitt 14-1 herrscht eine kontinuierliche Luftströmung, die zumindest im Bereich der Behälter in vertikaler Richtung von oben nach unten (in Richtung von der Decke zum Boden) gerichtet ist.

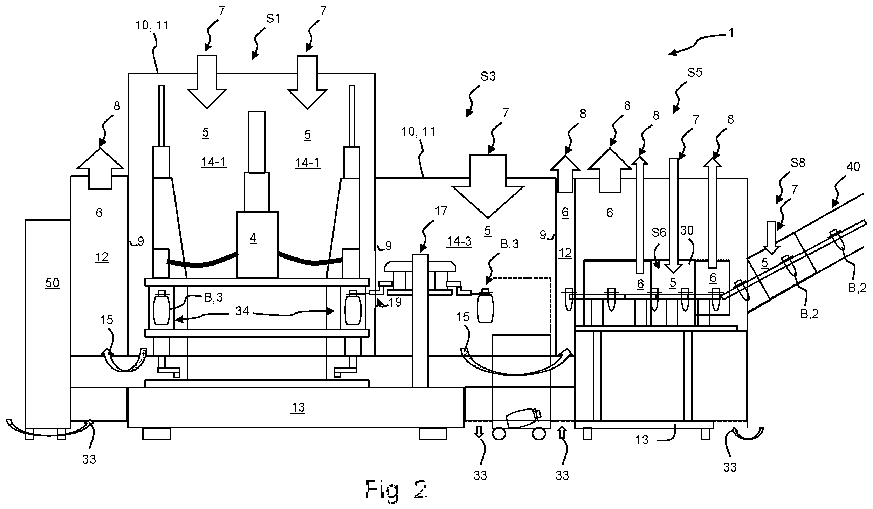

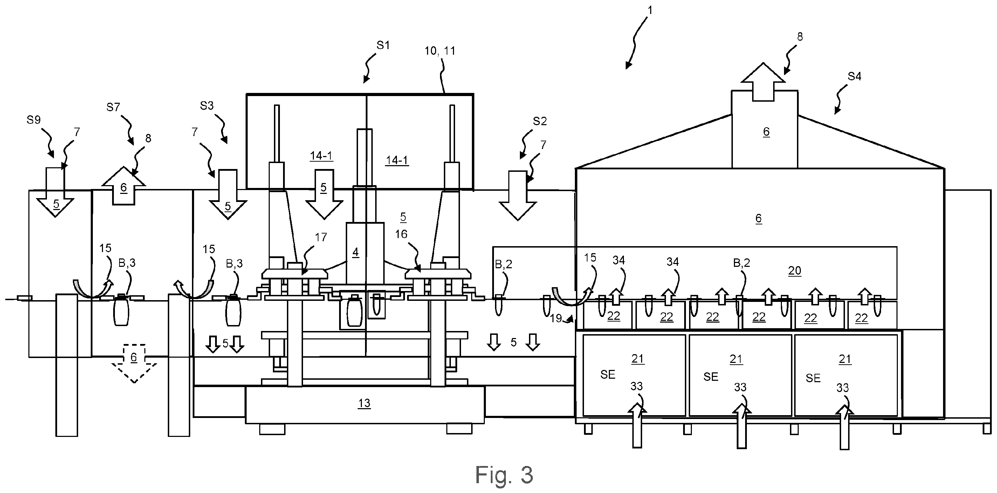

Die Trennwände 9 der ersten Sektion sind derart ausgeführt, dass sie die Behälter B auf deren Weg durch die erste Sektion S1 nach oben und unten überragen. Grundsätzlich wird dadurch gewährleistet, dass die Verdrängungsströmung 5 im Bereich der Behälter einheitlich in vertikaler Richtung von oben nach unten gerichtet ist. Dieser Aspekt wird weiter unten in Bezug auf die Figuren 2 und 3 noch näher erläutert. Nach oben sind die Trennwände 9 geschlossen. Im vorliegenden Fall bedeutet das, dass die Trennwände 9 der ersten Sektion mit der Decke des Maschinengehäuses 11 abschließen.

Die Trennwände der ersten Sektion sind abschnittweise von den Wänden 10 des Maschinengehäuses nach innen beabstandet. Dort bilden die Trennwände 9 gemeinsam mit den Wänden 10 des Maschinengehäuses 11 Kanäle (Räume, Schächte), sogenannten Absaugkanäle 12 der ersten Sektion, die nach oben und seitlich geschlossen sind. Insgesamt befinden sich die Trennwände 9 vorteilhaft innerhalb des von dem Maschinengehäuse 11 bzw. den Wänden 10 des Maschinengehäuses umhausten Bereiches.

An der Oberseite der Absaugkanäle 12 der ersten Sektion befinden sich Luftabführeinrichtungen 8, welche die Luft aus diesen Absaugkanälen 12 abführen und dadurch eine Saugströmung 6 für die Luft erzeugen.

Nach unten, also zum Boden 13, oder auch Aufstellboden des Maschinengehäuses 11 hin, schließen die Trennwände 9 der ersten Sektion nicht mit dem Boden 13 ab. Dort bleibt ein Bereich frei, durch den die Luft von dem inneren Luftverdrängungsabschnitt 14-1 nach außen zu den Absaugkanälen 12 der ersten Sektion fließen kann.

Die Luftströmungen entlang des Bodens (Aufstellboden) 13 und deren Richtung sind mit Pfeilen 15 stellenweise verdeutlicht.

Im hinteren Bereich (in Figur 1 oben) der Sektion 1 befindet sich noch ein elektrischer Schaltschrank 50, dessen Funktionsweise allgemein bekannt ist. Ebenfalls im hinteren Bereich befinden sich links und rechts des Absaugkanals 12 der ersten Sektion noch eine Luftwand 35 und eine Wasserwand 34 für die Luft- bzw. Wasserversorgung.

An die erste Sektion S1 können sich weitere Sektionen anschließen. Im vorliegenden Fall sind das eine zweite Sektion S2 und eine dritte Sektion S3.

Die Behandlungsmaschine 1 weist eine zweite Sektion S2 auf, die sich stromaufwärts der ersten Sektion S1 bezogen auf die Transportrichtung der Behälter B entlang des Transportpfades befindet.

In dieser zweiten Sektion S2 ist mindestens eine erste Transferreinrichtung 16, (Transferrad, Zuführeinrichtung, Zuführrad) mindestens teilweise aufgenommen. Diese Transferreinrichtung 16 dient dazu, die Behälter B, im vorliegenden Fall die Vorformlinge 2, der Behandlungseinrichtung 4 der ersten Sektion S1 zuzuführen. Die Transfereinrichtung 16 erstreckt sich zum Teil bis in die erste Sektion S1 hinein, was für den Transport der Behälter B,2 erforderlich ist. Die Übergabe der Behälter B,2 (Vorformlinge) an die Behandlungseinrichtung 4 erfolgt erst innerhalb der ersten Sektion S1.

Oberhalb der zweiten Sektion S2 ist eine Luftzuführeinrichtung 7 für sterile Luft 18 angeordnet. Mittels der Luftzuführeinrichtung 7 wird sterile Luft 18 relativ zentral von oben in die zweite Sektion S2 eingebracht.

In der zweiten Sektion S2 sind ebenfalls Trennwände 9 vorgesehen. Diese Trennwände 9 der zweiten Sektion umgeben die Transfereinrichtung 16 wenigstens abschnittweise. Zudem sind Trennwände 9 auch an den Übergängen zur ersten Sektion S1 und weiteren Sektionen S4 und S5 vorgesehen. Die weiteren Sektionen S4 und S5 werden nachfolgend genauer beschrieben.

Da sich die Behälter B generell entlang des Transportpfades stromabwärts von einer Sektion zu einer anderen Sektion bewegen, sind in den Trennwänden 9 zwischen benachbarten Sektionen, Durchbrechungen 19 vorgesehen, durch welche die Behälter B transportiert werden können. Typischerweise erstrecken sich Transporteinrichtungen durch die Durchbrechungen 19. Für diese Durchbrechungen ist bevorzugt, wenn diese mit einem geringen Spiel der durch die Durchbrechungen zu bewegenden Behälter bzw. Bereiche der Transfereinrichtungen formangepasst ausgeführt sind.

Durch die Trennwände 9 der zweiten Sektion und teilweise auch die Wände 10 des Maschinengehäuses 11 wird aufgrund der Luftzuführeinrichtung 7 in einem inneren Abschnitt der zweiten Sektion eine Verdrängungsströmung 5 erzeugt, der als zweiter innerer Luftverdrängungsabschnitt 14-2 in der zweiten Sektion S2 bezeichnet wird.

Die Trennwände 9 in der zweiten Sektion S2 sind ebenso ausgeführt wie in der ersten Sektion S1. Sie überragen die Behälter B nach oben und unten. Grundsätzlich wird dadurch gewährleistet, dass die Verdrängungsströmung 5 im Bereich der Behälter B,2 möglichst störungsfrei und einheitlich von oben nach unten gerichtet ist. Dadurch wird ein Vordringen von Keimen zu den Behältern und somit eine Verkeimung der Behälter möglichst effektiv verhindert. Nach oben sind die Trennwände 9 der zweiten Sektion geschlossen bzw. schließen mit dem Maschinengehäuse 11 ab.

Auch in der zweiten Sektion S2 sind die Trennwände 9 in einem Bereich von den Wänden 10 des Maschinengehäuses 11 nach innen beabstandet. Hier bilden die Trennwände 9 der zweiten Sektion ebenfalls gemeinsam mit den Wänden 10 des Maschinengehäuses 11 einen Absaugkanal 12 der zweiten Sektion, der nach oben geschlossen ist. An der Oberseite des Absaugkanals 12 der zweiten Sektion befindet sich eine Luftabführeinrichtung 8, welche die Luft aus diesem Absaugkanal 12 abführt und dadurch eine Saugströmung 6 für die Luft erzeugt.

Nach unten, also zum Boden 13 oder auch Aufstellboden des Maschinengehäuses 11 hin, schließen auch in der zweiten Sektion S2 die Trennwände 9 nicht mit dem Boden 13 ab. Dort bleibt ein Bereich frei, durch den die Luft von dem zweiten inneren Luftverdrängungsabschnitt 14-2 zu dem Absaugkanal 12 der zweiten Sektion fließen kann.

Auch in dem zweiten inneren Luftverdrängungsabschnitt 14-2 herrscht eine kontinuierliche Luftströmung in vertikaler Richtung von oben nach unten (von der Decke zum Boden).

Im Absaugkanal 12 der zweiten Sektion mit der Saugströmung 6 strömt die Luft in vertikaler Richtung von unten nach oben, also zum oberem Ende des Absaugkanals 12 der zweiten Sektion bzw. in Richtung der Luftabführeinrichtungen 8.

Die Behandlungsmaschine 1 weist eine dritte Sektion S3 auf, die sich stromabwärts der ersten Sektion S1 bezogen auf die Transportrichtung der Behälter B entlang des Transportpfades befindet.

In dieser dritten Sektion S3 ist eine zweite Transferreinrichtung 17 (Transferrad, Abführeinrichtung, Abführrad) teilweise aufgenommen. Diese Transferreinrichtung 17 dient dazu, die Behälter B, im vorliegenden Fall die fertigen Behälter 3, von der Behandlungseinrichtung 4 der ersten Sektion S1 abzuführen. Die Transfereinrichtung 17 erstreckt sich zum Teil bis in die erste Sektion S1 hinein, was für den Transport der Behälter B,3 erforderlich ist. Die Übergabe der Behälter B,3 (fertige Behälter) von der Behandlungseinrichtung 4 an die zweite Transfereinrichtung 17 erfolgt innerhalb der ersten Sektion S1.

Oberhalb der dritten Sektion S3 ist eine Luftzuführeinrichtung 7 für sterile Luft 18 angeordnet. Mittels der Luftzuführeinrichtung 7 wird sterile Luft 18 relativ zentral von oben in die dritte Sektion S3 eingebracht.

In der dritten Sektion S3 sind ebenfalls Trennwände 9 vorgesehen. Diese Trennwände 9 der dritten Sektion umgeben die Transfereinrichtung 17 wenigstens abschnittweise.

Es sind auch Trennwände 9 an den Übergängen zur ersten Sektion S1 und der siebten Sektion S7 vorgesehen.

Da sich die Behälter B generell entlang des Transportpfades stromabwärts von einer Sektion zu einer anderen Sektion bewegen, sind in den Trennwänden 9 zwischen den benachbarten Sektionen, Durchbrechungen 19 vorgesehen, durch welche die Behälter B transportiert werden können.

Durch die Trennwände 9 der dritten Sektion und teilweise auch die Wände 10 des Maschinengehäuses 11 wird aufgrund der Luftzuführeinrichtung 7 in einem inneren Abschnitt der dritten Sektion eine Verdrängungsströmung 5 erzeugt, die als dritter innerer Luftverdrängungsabschnitt 14-3 in der dritten Sektion S3 bezeichnet wird.

Die Trennwände 9 in der dritten Sektion S3 sind ebenso ausgeführt wie in der ersten und zweiten Sektion S1. Sie überragen die Behälter B nach oben und unten. Grundsätzlich wird dadurch gewährleistet, dass die Verdrängungsströmung 5 im Bereich der Behälter B,2 möglichst störungsfrei und einheitlich von oben nach unten gerichtet ist. Dadurch wird eine Verkeimung der Behälter B auch hier möglichst effektiv verhindert. Nach oben sind die Trennwände 9 der dritten Sektion geschlossen bzw. schließen mit dem Maschinengehäuse 11 ab.

Auch in der dritten Sektion S3 sind die Trennwände 9 in einem Bereich von den Wänden 10 des Maschinengehäuses 11 nach innen beabstandet. Hier bilden die Trennwände 9 der dritten Sektion ebenfalls gemeinsam mit den Wänden 10 des Maschinengehäuses 11 einen Absaugkanal 12 der dritten Sektion, der nach oben geschlossen ist. An der Oberseite des Absaugkanals 12 der dritten Sektion befindet sich eine Luftabführeinrichtung 8, welche die Luft aus dem besagten Absaugkanal 12 abführt und dadurch eine Saugströmung 6 für die Luft erzeugt.

Nach unten, also zum Boden 13 oder auch Aufstellboden des Maschinengehäuses 11 hin, schließen auch in der dritten Sektion S3 die Trennwände 9 der dritten Sektion nicht mit dem Boden 13 ab. Dort bleibt ein Bereich frei, durch den die Luft 15 von dem zweiten inneren Luftverdrängungsabschnitt 14-3 zu dem Absaugkanal 12 der dritten Sektion fließen kann.

Auch in dem dritten inneren Luftverdrängungsabschnitt 14-3 herrscht eine kontinuierliche Luftströmung in vertikaler Richtung von oben nach unten (von der Decke zum Boden).

Im Absaugkanal 12 der dritten Sektion mit der Saugströmung 6 strömt die Luft in vertikaler Richtung von unten nach oben, also zum oberem Ende des Absaugkanals 12 bzw. in Richtung der Luftabführeinrichtungen 8.

Die Behandlungsmaschine 1 weist eine vierte Sektion S4 auf, die sich stromaufwärts der zweiten Sektion S2 bezogen auf die Transportrichtung der Behälter B entlang des Transportpfades befindet.

Zwischen der zweiten Sektion S2 und der vierten Sektion S4 befinden sich ebenfalls Trennwände 9 der vierten Sektion mit Durchbrechungen 19. Durch diese Durchbrechungen 19 können die Behälter B zwischen der zweiten Sektion S2 und der vierten Sektion S4 hin- und hertransportiert werden.

In der vierten Sektion S4 ist eine Heizvorrichtung 20 angeordnet. Mittels der Heizvorrichtung 20 können Vorformlinge 2 auf eine Umformtemperatur erhitzt werden. Die Funktionsweise und der Aufbau einer solchen Heizvorrichtung 20 ist grundsätzlich bekannt.

Die Vorformlinge 2 werden von der zweiten Sektion S2 aus an die vierte Sektion S4 und somit an die Heizvorrichtung 20 übergeben. Nach dem Erwärmen durch die Heizvorrichtung 20 werden die Vorformlinge 2 wieder von der vierten Sektion S4 an die zweite Sektion S2 übergeben.

Oberhalb der vierten Sektion S4 ist eine Luftabführeinrichtung 8 angeordnet. Diese sorgt für eine Saugströmung 6 innerhalb der vierten Sektion S4.

Die Behandlungsmaschine 1 weist eine fünfte Sektion S5 auf, die sich stromaufwärts der zweiten Sektion S2 und vierten Sektion S4 bezogen auf die Transportrichtung der Behälter B entlang des Transportpfades befindet.

In der fünften Sektion S5 befinden sich mehrere Transfereinrichtungen, insbesondere eine vierte, fünfte und sechste Transfereinrichtung, 25, 26, 27, 28 bzw. ein viertes, fünftes, sechstes und siebtes Transferrad 25, 26, 27, 28.

Um die Transferreinrichtungen 25, 26, 27, 28 sind ebenfalls Trennwände 9 der fünften Sektion angeordnet. Allerdings schließen diese Trennwände 9 im Unterschied zu den Trennwänden 9 der Sektionen S1 bis S4 hier mit einer undurchlässigen Bodenplatte nach unten ab. Die Trennwände 9 in der fünften Sektion S5 sind also nach unten anders ausgeführt als in der ersten, zweiten und dritten Sektion S1, S2, S3. Nach oben und unten überragen die Trennwände die Behälter B. Nach oben sind die Trennwände 9 der fünften Sektion geschlossen bzw. schließen mit dem Maschinengehäuse 11 ab.

Oberhalb der fünften Sektion S5 sind mehrere Luftabführeinrichtungen 8 angeordnet. Diese bewirken eine Saugströmung 6 innerhalb der fünften Sektion S5.

Durch die Trennwände 9 der fünften Sektion ist die fünfte Sektion S5 in innere Abschnitte 31 und äußere Abschnitte 32 unterteilt, in denen unterschiedlich starke Saugströmungen 6 wirken.

In einem inneren Saugströmungsabschnitt 31 innerhalb der Trennwände 9 der fünften Sektion herrscht eine Saugströmung VD. In einem äußeren Saugströmungsabschnitt der fünften Sektion zwischen den Trennwänden 31 der fünften Sektion und den Wänden 10 des Maschinengehäuses herrscht eine Saugströmung VC. Dabei ist die Saugströmung VD größer als die Saugströmung VC. Dadurch wird auch in der fünften Sektion S5 eine vorteilhafte Vorzugsströmungsrichtung von den Bereichen mit der Saugströmung VC zu den Bereichen mit einer Saugströmung VD erzeugt.

Innerhalb der fünften Sektion S5 kann eine sechste Sektion S6 eingerichtet sein. In der sechsten Sektion S6 befindet sich eine Beaufschlagungseinrichtung für Sterilisierfluid 30, in dem die Behälter B, in diesem Fall die Vorformlinge 2, mit Sterilisierfluid beaufschlagt werden. Die sechste Sektion S6 ist durch Trennwände 9 von dem übrigen Bereich der fünften Sektion S5 getrennt. Die Trennwände 9 der sechsten Sektion bilden einen inneren Abschnitt der sechsten Sektion. Oberhalb dieses inneren Abschnitts der sechsten Sektion ist eine Luftzuführeinrichtung 7 angeordnet. Dadurch herrscht in dem inneren Abschnitt der sechsten Sektion S6 eine Verdrängungsströmung 5. Dementsprechend kann hier ein innerer (Luft-)Verdrängungsabschnitt 33 innerhalb der fünften Sektion S5 eingerichtet sein. In dem inneren Luftverdrängungsabschnitt 33 befindet sich die Beaufschlagungseinrichtung für Sterilisierfluid 30. Auch die sechste Sektion S6 ist mittels der Trennwände 9 und die Bodenplatte fest nach untern verschlossen. Die Luft kann damit nur durch Durchbrechungen der Trennwände von der sechsten Sektion S6 zur fünften Sektion S5 gelangen. Zudem gelangt die Luft von der zweiten Sektion S2 und der achten Sektion S8 ebenfalls durch die Durchbrechungen für den Transport der Vorformlinge B,2 zur fünften Sektion S5.

Die Behandlungsmaschine 1 umfasst eine siebte Sektion S7, die zwei weitere Transfereinrichtungen 24, 29 teilweise umgibt, wobei die siebte Sektion S7 stromabwärts (bezüglich des Transportpfades der Behälter) der dritten Sektion S3 angeordnet ist. Mindestens eine Transfereinrichtung 24 kann sich von der dritten Sektion S3 in die siebte Sektion S7 erstrecken bzw. teils in der dritten S3 und teils in der siebten Sektion S7 angeordnet sein.

Die siebte Sektion S7 kann auch als Unterdruckschleuse bezeichnet werden, durch welche die Behälter B,3 abtransportiert werden.

Weitere Trennwände 9 sind zwischen der siebten Sektion S7 und der dritten Sektion S3 angeordnet, wobei diese Trennwände S9 mit dem Boden 13 abschließen und zusätzlich Durchbrechungen 19 (für die Transfereinrichtung 24) aufweisen, durch welche die Behälter B,3 mit der Transfereinrichtung 24 von der dritten Sektion S3 zur siebten Sektion S7 transportiert werden können.

In einer Ausgestaltung kann eine Luftabführeinrichtung 8 an die siebte Sektion S7 von oben oder von unten angeschlossen sein. Somit kann Luft aus der siebten Sektion S7 mittels der Luftabführeinrichtung 8 abgesaugt werden, so dass in der siebten Sektion S7 eine von unten nach oben oder von oben nach unten gerichtet Saugströmung 6 eingerichtet wird, welche die Transfereinrichtungen 24, 29 teilweise umströmt, wobei die Luft 15 ebenfalls von der dritten Sektion S3 zur siebten Sektion S7 durch die Durchbrechungen der Trennwände 9 für den Transport der Behälter B,3 fließt.

Die Behandlungsmaschine 1 kann eine achte Sektion S8 umfassen, in der mindestens eine Zuführeinrichtung 40 für Behälter B, insbesondere Vorformlinge 2 angeordnet ist.

Die achte Sektion S8 ist stromaufwärts (bezüglich der Transportrichtung der Behälter B,2) der fünften Sektion S5 angeordnet. Zwischen der achten Sektion S8 und der fünften Sektion S5 sind Trennwände angeordnet, wobei diese Trennwände 9 auch hier Durchbrechungen19 aufweisen, durch welche die Behälter B,2 von der achten Sektion S8 zur fünften Sektion S5 transportiert werden können.

Eine Luftabführeinrichtung 8 ist von oben an die achte Sektion S8 angeschlossen, um Luft aus der achten Sektion S8 mittels der Luftabführeinrichtung 8 abzusaugen, so dass in der achten Sektion S8 eine von unten nach oben gerichtet Saugströmung 6 eingerichtet wird.

Wie zuvor dargestellt herrschen in den Sektionen S1-S8 jeweils abschnittweise individuelle Saugströmungen SC und individuelle Verdrängungsströmungen VA, die in Figur 1 gemäß den nachfolgenden Erläuterungen eingetragen sind.

In der ersten, zweiten und dritten Sektion S1, S2 und S3 herrscht in den inneren Verdrängungsabschnitten 14-1, 14-2, 14-3 jeweils eine Verdrängungsströmung einer individuellen Größe VA1, VA2 und VA3. In den Absaugkanälen 12 herrscht jeweils eine Saugströmung mit einer individuellen beispielhaften Größe SCx, SCy, SCz etc.

Die Sektionen und Bereiche mit einer Saugströmung SC sind neben den Absaugkanälen 12 auch die vierte Sektion S4 und die siebte Sektion S7. Auch in diesen herrschen individuelle Saugströmungen der Größe SC4 und SC7. In der fünften Sektion S5 herrscht im äußeren Saugströmungsabschnitt 32 (also außerhalb der Trennwände 9) ebenfalls eine individuelle Saugströmung SC5. Nur in der sechsten Sektion S6, die einen Unterabschnitt der fünften Sektion S5 darstellt, herrschte eine individuelle Verdrängungsströmung VA6. In den inneren Saugströmungsabschnitten 31 der fünften Sektion S5 herrschte eine Saugströmung SC5i, die wiederum größer sein kann als die Saugströmung SC5a im äußeren Saugströmungsabschnitt 32. In der achten Sektion S8, also im Bereich der Zuführung 40 kurz vor dem Eintritt in die achte Sektion S8 herrscht eine Verdrängungsströmung der Größe VA8.

Eine Saugströmung SC4 herrscht im Bereich der Ansaugfilter 21 in der vierten Sektion S4 (bei der Heizvorrichtung) und in der siebten Sektion S7 wird mit einer Saugstärke von SC7 gearbeitet

Insgesamt wird durch die vorliegende Offenbarung ein Verfahren und eine Vorrichtung bereitgestellt, bei denen innere Verdrängungsabschnitte von Absaugabschnitten umgeben sind. Ferner sind Sektionen, wie S1, S2, S3, S6, und S8 die eine Verdrängungsströmung aufweisen, von Sektionen S4, S7, S5 umgeben, die eine Saugströmung aufweisen. Zudem gibt es die Absaugkanäle 12, welche die Sektionen S1, S2 und S3 umgeben und individuelle Saugströmungen SCx, SC, SCz etc. haben. Diese Abstimmung ermöglicht einen hochgradigen kontaminationsfreien Transport (einen kontaminationsfreien Transportpfad) und Behandlung der Behälter B innerhalb der Behandlungsmaschine 1. Allgemein wird so erreicht, dass im Inneren (Sektionen S1, S2, S3) in den als besonders keimkritisch angesehenen Bereichen (besonders die Behandlungseinrichtung 4 bzw. Behandlungsrad) ein Überdruck entsteht und eine Verdrängungsströmung etwaige Keime bzw. Mikroorganismen von den Behältern B fernhält. Durch das Umgeben der "inneren Bereiche" 14-1, 14-2, 14-3 mit äußeren Absaugkanälen 12 erfolgt eine gezielte Strömungsführung radial nach außen, ebenfalls weg von der Behandlungseinrichtung 4 und den Behältern B. Deshalb wird in den besonders kritischen Sektionen S1, S2, S3 (Verdrängungsabschnitte 14-1, 14-2, 14-3) Luft zugeführt und über die umgebenden äußeren Absaugkanälen 12 nach außen abgeführt.

Fig. 2 ist eine vereinfachte schematische seitliche Ansicht der Behandlungsmaschine 1 bzw. Vorrichtung zum Behandeln von Behältern 1 unter Sterilbedingungen aus Fig.1. In dieser Darstellung sind die erste Sektion S1, die dritte Sektion S3, die fünfte Sektion S5, die sechste Sektion S6 und die achte Sektion S8 mit Zuführeinrichtung 40 für die Behälter (Vorformlinge) B,2 von der Seite in einer Art Schnittdarstellung dargestellt. In dieser Darstellung sind die Absaugkanäle 12 mit den Trennwänden 9 hervorzuheben. Je ein Absaugkanal 12 ist links der ersten Sektion S1 und rechts der dritten Sektion S3 angeordnet. Die Luft fließt hier entlang der Pfeile 15 unter den Trennwänden 9 hindurch jeweils von dem ersten inneren Verdrängungsabschnitt 14-1 zum linken Absaugkanal 12 und von dem dritten inneren Verdrängungsabschnitt 14-3 zum rechten Absaugkanal 12. Unterhalb des Bodens bzw. Aufstellbodens 13 sind durch Pfeile noch Luftströmungen von Hallenluft 33 zu sehen. Die Größe der Pfeile indiziert nicht die Strömungsstärken. Das Maschinengehäuse 11 muss vorteilhaft von unten nicht luftdicht sein. Hier kann von außen Hallenluft unterhalb der Sektionen eintreten. Aufgrund der Strömungsverhältnisse und Trennwände 9 sowie dem Zuführen steriler Luft dringen die Hallenluft 33 und darin enthaltene Keime bzw. Mikroorganismen nicht bis zu den Behältern B vor.

Fig. 3 ist eine weitere vereinfachte schematische seitliche Ansicht der Vorrichtung zum Behandeln von Behältern unter Sterilbedingungen aus Fig.1. In dieser Darstellung sind die erste Sektion S1, die zweite Sektion S2, die dritte Sektion S3, die vierte Sektion S4, die siebte Sektion S7 und die neunte Sektion S9 von der Seite in einer Art Schnittdarstellung dargestellt.

Wie bereits beschrieben ist in der vierten Sektion S4 eine Saugströmung 6 eingerichtet. Dabei wird Hallenluft 33 seitlich angesaugt und durch Filter 21 (bspw. HEPA-Filter) geleitet, bevor sie als sterile Kühlluft 34 durch die Heizeinrichtungen 22 der Heizvorrichtung strömt und von hier in vertikaler Richtung weiter nach oben zur Luftabführeinrichtung 8 gelangt. Im Bereich der Filter 21 liegt eine Saugströmung SE vor.

Ferner gelangt Luft 15 von der zweiten Sektion S2 durch die Durchbrechungen 19 in den Trennwänden 9 zwischen zweiter Sektion S2 und vierter Sektion S4 zur vierten Sektion S4 und wird dort ebenfalls in vertikaler Richtung nach oben gesaugt. Ähnlich verhält sich dies auch bei der dritten, siebten und neunten Sektion S3, S7, S9. Auch wird durch die Pfeile 15 verdeutlicht, wie Luft durch die Durchbrechungen 19 in den Trennwänden 9 zwischen den Sektionen fließt. Dabei ist eine Besonderheit, dass die Strömung in der siebten Sektion S7 von Saugströmung 6 auf Verdrängungsströmung 5 (und umgekehrt) umgestellt werden kann. Dadurch ändert sich die Strömungsrichtung 15 zwischen den Sektionen.

Die Trennwände 9 können vorteilhaft luftdicht und starr sein. Sie können aus Kunststoff oder Metall bestehen. Vorteilhaft können die Trennwände 9 auch - wenigstens in bestimmten Sektionen oder Bereichen von Sektionen - aus transparentem Kunststoff bestehen. Das ermöglicht zusätzlich einen besseren Überblick. Insbesondere können Wände aus transparentem Kunststoff als Trennwände 9 im Bereich der ersten Sektion S1, der zweiten Sektion S2 und dritten Sektion S3 eingesetzt werden. Dort können die Trennwände 9 aus transparentem Kunststoff vorteilhaft zwischen den Sektionen eingesetzt werden.

### Bezugszeichenliste

- 1: Vorrichtung zum Behandeln von Behältern, Behandlungsmaschine
- 2: Vorformling
- 3: fertiger umgeformter Behälter
- 4: Behandlungseinrichtung, Behandlungsrad, Umformeinrichtung, Umformrad
- 5: (Luft-)Verdrängungsströmung
- 6: (Luft-)Saugströmung
- 7: Luftzuführeinrichtung für sterile Luft, Sterilluftzuführeinrichtung
- 8: Luftabführeinrichtung
- 9: Trennwand, Trennwände
- 10: Wand des Maschinengehäuses 11
- 11: Maschinengehäuse
- 12: Absaugkanal, Absaugschacht
- 13: Boden, Aufstellboden
- 14: (innerer) Luftverdrängungsabschnitt
- 15: Pfeil für Luftströmung; Richtung der Luftströmung
- 16: Erste Transfereinrichtung, Transferrad, Zuführeinrichtung, Zuführrad
- 17: Zweite Transfereinrichtung, Transferrad, Abführeinrichtung, Abführrad
- 18: Sterile Luft
- 19: Durchbrechung der Trennwand 9
- 20: Heizvorrichtung
- 21: Filter, Luftfilter
- 22: Heizeinrichtung, Heizkasten
- 23: Dritte Transfereinrichtung, Transferrad
- 24: Vierte Transfereinrichtung, Transferrad
- 25: Fünfte Transfereinrichtung, Transferrad
- 26: Sechste Transfereinrichtung, Transferrad
- 27: Siebte Transfereinrichtung, Transferrad
- 28: Achte Transfereinrichtung, Transferrad
- 29: Neunte Transfereinrichtung, Transferrad
- 30: Beaufschlagungseinrichtung für Sterilisierfluid
- 31: innerer Saugströmungsabschnitt in der fünften Sektion S5
- 32: äußerer Saugströmungsabschnitt in der fünften Sektion S5
- 33: Hallenluft, Pfeil für Hallenluft
- 34: Wasserwand
- 35: Luftwand
- 40: Zuführung für Behälter bzw. Vorformlinge, Schiene, Rutsche, Luftförderer
- 50: Schaltschrank, elektrisch
- B: Behälter (alle Arten, fertige Behälter, Vorformlinge etc.)
- S1: Sektion 1, erste Sektion, Sektion mit Behandlungseinrichtung
- S2: Sektion 2, zweite Sektion, Sektion mit einer oder mehreren Transfereinrichtungen (Transferrad, Zuführeinrichtung, Zuführrad)
- S3: Sektion 3, dritte Sektion, Sektion mit einer oder mehreren Transfereinrichtungen (Transferrad, Abführrad)
- S4: Sektion 4, vierte Sektion, Sektion mit Heizvorrichtung
- S5: Sektion 5, fünfte Sektion, Sektion mit einem oder mehreren Transfereinrichtungen bspw. Vereinzelungsrad
- S6: Sektion 6, sechste Sektion, Sektion mit einer Beaufschlagungseinrichtung für Sterilisierfluid
- S7: Sektion 7, siebte Sektion, Sektion mit einem oder mehreren Transferreinrichtungen bspw. Transferräder, Abführeinrichtung, Abführrad
- S8: Sektion 8, achte Sektion, Sektion als Teil der Zuführung für Behälter 40
- VA: Größe einer Luft-Verdrängungsströmung
- SC: Größe einer Luft-Saugströmung
- SE: Größe der Luft-Saugströmung in Sektion S4

## Patentansprüche

1. Verfahren zur kontaminationsfreien Behandlung von Behältern in einer Behandlungsmaschine (1), die von einem über einem Aufstellboden (13) stehenden Maschinengehäuse (11) umgeben ist, wobei die Behandlungsmaschine (1) mindestens eine erste Sektion (S1) umfasst in der eine Behandlungseinrichtung (4) für die Behälter, insbesondere ein Behandlungsrad angeordnet ist, wobei das Verfahren umfasst: Anordnen von Trennwänden (9) in der ersten Sektion (S1) und mindestens abschnittweises Anordnen der Trennwände um die Behandlungseinrichtung (4) herum zur Ausbildung eines von den Trennwänden (9) mindestens teilweise umgebenen inneren Abschnittes, Anschließen einer Luftzuführeinrichtung (7) für sterile Luft an den inneren Abschnitt und Zuführen von steriler Luft innerhalb des von den Trennwänden (9) umschlossenen inneren Abschnittes, derart dass mindestens im Bereich der Behälter (B) ein erster innerer Luftverdrängungsabschnitt (14) ausgebildet wird, , wobei die Trennwände (9) mindestens abschnittweise vom Maschinengehäuse (11), bevorzugt nach innen, beabstandet angeordnet sind, so dass zwischen dem Maschinengehäuse (11) und den Trennwänden (9) äußere Absaugkanäle (12) gebildet werden, welche die Behandlungseinrichtung (4) zu einem überwiegenden Teil umgeben, wobei sich die Trennwände (9) mindestens abschnittweise entlang eines Transportpfades der Behälter (B) um die Behandlungseinrichtung (4) erstrecken und die Behälter (B) in vertikaler Richtung nach oben und unten überragen, ohne mit dem Boden (13), insbesondere Aufstellboden, abzuschließen, wobei die Absaugkanäle (12) seitlich und nach oben geschlossen sind und durch das Zuführen steriler Luft in dem ersten inneren Luftverdrängungsabschnitt (14) eine zumindest im Bereich der Behälter (B) vertikale Verdrängungsströmung (5) gebildet wird, Anschließen der äußeren Absaugkanäle (12) an eine Luftabführeinrichtung (8), Absaugen der Luft aus den äußeren Absaugkanälen (12) mittels der Luftabführeinrichtung (8), so dass eine Saugströmung in den Absaugkanälen (12) gebildet wird und Luft vom ersten inneren Luftverdrängungsabschnitt (14) in radialer Richtung zu den äußeren Absaugkanälen (12) unter den Trennwänden (9) hindurch vorbeifließt.

2. Verfahren nach Anspruch 1, wobei die Behandlungsmaschine (11) eine zweite Sektion (S2) umfasst und eine erste Transfereinrichtung (16), insbesondere ein Zuführrad für Behälter (B,2), zumindest teilweise in der zweiten Sektion (S2) aufgenommen ist und das Verfahren umfasst: Anordnen von Trennwänden (9) in und entlang der zweiten Sektion (S2) und mindestens abschnittweise um die Transfereinrichtung (16) herum zur Ausbildung eines von diesen Trennwänden (9) der zweiten Sektion (S2) mindestens teilweise umgebenen inneren Abschnittes der zweiten Sektion (S2), Anschließen einer Luftzuführeinrichtung (7) für sterile Luft an den inneren Abschnitt der zweiten Sektion (S2) und Zuführen von steriler Luft des von den Trennwänden (9) umschlossenen inneren Abschnittes der zweiten Sektion (S2), derart dass ein zweiter innerer Luftverdrängungsabschnitt (14) ausgebildet wird, wobei die Trennwände (9) der zweiten Sektion (S2) mindestens abschnittweise vom Maschinengehäuse (11), bevorzugt nach innen, beabstandet angeordnet sind, so dass zwischen dem Maschinengehäuse (11) und den Trennwänden (9) der zweiten Sektion (S2) mindestens ein äußerer Absaugkanal (12) der zweiten Sektion (S2) gebildet wird, wobei die Trennwände (9) der zweiten Sektion (S2) die Behälter (B) in vertikaler Richtung nach oben und unten überragen, ohne mit dem Boden (13), insbesondere Aufstellboden, abzuschließen, wobei der Absaugkanal (12) der zweiten Sektion (S2) seitlich und nach oben geschlossen ist und durch das Zuführen steriler Luft in dem zweiten inneren Luftverdrängungsabschnitt (14-2) eine zumindest im Bereich der Behälter (B) vertikale Verdrängungsströmung (5) gebildet wird, Anschließen des äußeren Absaugkanals (12) der zweiten Sektion (S2) an eine Luftabführeinrichtung (8), Absaugen der Luft aus dem äußeren Absaugkanal (12) der zweiten Sektion (S2) mittels der Luftabführeinrichtung (8), so dass eine Saugströmung in dem Absaugkanal (12) der zweiten Sektion (S2) gebildet wird und, insbesondere Luft vom ersten inneren Luftverdrängungsabschnitt (14-1) zum zweiten Luftverdrängungsabschnitt (14-2), und vom zweiten Luftverdrängungsabschnitt in radialer Richtung zu dem äußeren Absaugkanal (12) der zweiten Sektion (S2) unter den Trennwänden (9) der zweiten Sektion (S2) hindurch vorbeifließt.

3. Verfahren nach Anspruch 2, wobei zwischen der ersten Sektion (S1) und der zweiten Sektion (S2) Trennwände (9) angeordnet sind, die nicht mit dem Boden (11) abschließen und zusätzlich Durchbrechungen (19) aufweisen, durch welche die Behälter (B,2) von der zweiten Sektion (S2) zur ersten Sektion (S1) transportiert werden können.

4. Verfahren nach einem der Ansprüche 1 bis 3, wobei die Behandlungsmaschine (11) eine dritte Sektion (S3) umfasst, in der eine zweite Transfereinrichtung (17), insbesondere ein Abführrad für Behälter (B,3), zumindest teilweise aufgenommen ist, wobei das Verfahren umfasst: Anordnen von Trennwänden (9) in und entlang der dritten Sektion (S3) und mindestens abschnittweise um die Transfereinrichtung (16) herum zur Ausbildung eines von diesen Trennwänden (9) der dritten Sektion (S3) mindestens teilweise umgebenen inneren Abschnittes der dritten Sektion (S3), Anschließen einer Luftzuführeinrichtung (7) für sterile Luft an den inneren Abschnitt der dritten Sektion (S3) und Zuführen von steriler Luft innerhalb des von den Trennwänden (9) der dritten Sektion (S3) umschlossenen inneren Abschnittes der dritten Sektion (S3), derart dass ein dritter innerer Luftverdrängungsabschnitt (14) ausgebildet wird, , wobei die Trennwände (9) der dritten Sektion (S3) mindestens abschnittweise vom Maschinengehäuse (11), bevorzugt nach innen, beabstandet angeordnet sind, so dass zwischen dem Maschinengehäuse (11) und den Trennwänden (9) der dritten Sektion (S3) mindestens ein äußerer Absaugkanal (12) der dritten Sektion (S3) gebildet wird, wobei die Trennwände (9) der dritten Sektion (S3) die Behälter (B) in vertikaler Richtung nach oben und unten überragen, ohne mit dem Boden (13), insbesondere Aufstellboden, abzuschließen, wobei der Absaugkanal (12) der dritten Sektion (S3) seitlich und nach oben geschlossen ist und durch das Zuführen steriler Luft in dem zweiten inneren Luftverdrängungsabschnitt (14) eine zumindest im Bereich der Behälter (B) von oben nach unten gerichtete vertikale Verdrängungsströmung (5) gebildet wird, Anschließen des äußeren Absaugkanals (12) der dritten Sektion (S3) an eine Luftabführeinrichtung (8), Absaugen der Luft aus dem äußeren Absaugkanal (12) der dritten Sektion (S3) mittels der Luftabführeinrichtung (8), so dass eine Saugströmung in dem Absaugkanal (12) der dritten Sektion (S3) gebildet wird und Luft, insbesondere vom ersten inneren Luftverdrängungsabschnitt (14-1) zum dritten Luftverdrängungsabschnitt (14-3), und vom dritten Luftverdrängungsabschnitt (14-3) in radialer Richtung zu dem äußeren Absaugkanal (12) der dritten Sektion (S3) unter den Trennwänden (9) hindurch vorbeifließt.

5. Verfahren nach Anspruch 4, wobei zwischen der ersten Sektion (S1) und der dritten Sektion (S2) Trennwände (9) angeordnet sind, die nicht mit dem Boden (11) abschließen und zusätzlich Durchbrechungen (19) aufweisen, durch welche die Behälter (B,3) von der ersten Sektion (S1) zur dritten Sektion (S3) transportiert werden können.

6. Verfahren nach einem der vorstehenden Ansprüche, wobei mindestens eine der Luftzuführeinrichtungen (7) seitlich oberhalb der Behälter (B) und/oder von oben oberhalb der Behälter (B) und/oder von oben von einer Decke an den jeweiligen inneren Luftverdrängungsabschnitt (14-1, 14-2, 14-3) der ersten, zweiten und/oder dritte Sektion (S1, S2, S3) angeschlossen ist und mindestens eine Luftabführeinrichtung (8) seitlich und/oder von oben an einen der Absaugkanäle (12) angeschlossen ist.

7. Verfahren nach einem der Ansprüche 2 und 3 bis 6 soweit sich diese auf Anspruch 2 zurückbeziehen, wobei die Behandlungsmaschine (11) eine vierte Sektion (S4) umfasst, in der eine Heizvorrichtung (20) angeordnet ist, wobei das Verfahren umfasst: Anordnen von Trennwänden (9) zwischen der vierten Sektion (S4) und der zweiten Sektion (S2), wobei diese Trennwände (9) der vierten Sektion (S4) mit dem Boden (11) abschließen und Durchbrechungen (19) aufweisen, durch welche die Behälter (B,2; B,3) von der vierten Sektion (S4) zur zweiten Sektion (S2) transportiert werden können und umgekehrt, Anschließen einer Luftabführeinrichtung (8) an die vierte Sektion (S4) von oben und Absaugen der Luft (18) aus der vierten Sektion (S4) mittels der Luftabführeinrichtung (8), derart dass in der vierten Sektion (S4) eine von unten nach oben gerichtet Saugströmung (6) ausgebildet wird, welche die Heizvorrichtung (20) durchströmt, wobei die Luft (18) ebenfalls von der zweiten Sektion (S2) zur vierten Sektion (S4) durch die Durchbrechungen (19) hindurch fließt.

8. Verfahren nach einem der Ansprüche 3 und 4 bis 7 soweit auf Anspruch 2 zurückbezogen, wobei die Behandlungsmaschine (11) eine fünfte Sektion (S5) umfasst, in der weitere Transfereinrichtungen (23, 24, 25) zumindest teilweise angeordnet sind, wobei das Verfahren umfasst: Anordnen von Trennwänden (9) zwischen der fünften Sektion (S5) und der zweiten Sektion (S2), wobei diese Trennwände (9) der fünften Sektion (S5) mit dem Boden (11), insbesondere einem Zwischenboden (11), abschließen und Durchbrechungen (19) aufweisen, durch welche die Behälter (B,2) von der fünften Sektion (S5) zur zweiten Sektion (S2) transportiert werden können, Anordnen von Trennwänden (9) innerhalb der fünften Sektion (S5) und um die weiteren Transfereinrichtungen (23, 24, 25) herum, wobei diese weiteren Trennwände (9) der fünften Sektion (S5) mindestens abschnittweise vom Maschinengehäuse (11) beabstandet angeordnet sind, wobei die Trennwände (9) der fünften Sektion (S5) die Behälter (B) in vertikaler Richtung nach oben und unten überragen, und mit dem Boden (13) abschließen, Anschließen der inneren Abschnitte (31) der fünften Sektion (S5) von oben an eine Luftabführeinrichtung (8), Absaugen der Luft aus den inneren Abschnitten (31) der fünften Sektion (S5) mittels der Luftabführeinrichtung (8), so dass innere Saugströmungsabschnitte (31) gebildet werden, in denen eine von unten nach oben gerichtete Saugströmung (6) herrscht.

9. Verfahren nach Anspruch 8, wobei zwischen dem Maschinengehäuse (11) und den Trennwänden (9) der fünften Sektion (S5) äußere Saugströmungsabschnitte (32) gebildet werden, in denen eine Saugströmung herrscht, deren Stärke geringer ist als die Stärke der Saugströmung innerhalb der inneren Saugströmungsabschnitte (31) der fünften Sektion (S5).

10. Verfahren nach einem der vorstehenden Ansprüche, wobei die Behandlungsmaschine (11) eine sechste Sektion (S6) umfasst, in welcher eine Beaufschlagungseinrichtung für Sterilisierfluid (30) angeordnet ist, die die Behälter mit einem Sterilisierfluid beaufschlagt.

11. Verfahren nach Anspruch 10, wobei die sechste Sektion (S6) innerhalb der fünften Sektion (S5) durch Anordnen von Trennwänden (9) um die Beaufschlagungseinrichtung für Sterilisierfluid (30) herum gebildet wird, wobei diese Trennwände (9) der sechsten Sektion (S6) die Behälter (B,2) nach oben und unten überragen und mit dem Boden, insbesondere Zwischenboden, abschließen und Anschließen einer Luftzuführeinrichtung (7) von oben an die sechste Sektion (S6), derart dass in der sechsten Sektion (S6) eine vertikale von oben nach unten gerichtete Verdrängungsströmung (5) herrscht.

12. Verfahren nach Anspruch 4 und 5 bis 11, soweit auf Anspruch 4 zurückbezogen, wobei in dem Maschinengehäuse (11) eine siebte Sektion (S7) vorgesehen ist, die mindestens eine weitere Transfereinrichtung teilweise umgibt, wobei die siebte Sektion (S7) bezüglich des Transportpfades der Behälter (B,3) stromabwärts der dritten Sektion (S3) angeordnet ist und sich mindestens eine Transfereinrichtung von der dritten Sektion (S3) in die siebte Sektion (S7) erstreckt, Anordnen von Trennwänden (9) zwischen der siebten Sektion (S7) und der dritten Sektion (S3), wobei diese Trennwände (9) der siebten Sektion (S7) mit dem Boden (11) abschließen und Durchbrechungen (19) aufweisen, durch welche die Behälter (B,3) mit der Transfereinrichtung von der dritten Sektion (S3) zur siebten Sektion (S7) transportiert werden können, Anschließen einer Luftabführeinrichtung (8) an die siebte Sektion (S7) von oben oder von unten und Absaugen der Luft aus der siebten Sektion (S7) mittels der Luftabführeinrichtung (8), derart dass in der siebten Sektion (S7) eine Saugströmung (6) ausgebildet wird, welche die Transfereinrichtung umströmt, wobei die Luft ebenfalls von der dritten Sektion (S3) zur siebten Sektion (S7) durch die Durchbrechungen (19) hindurch fließt.

13. Verfahren nach Anspruch 8 und 9 bis 12 soweit auf Anspruch 8 zurückbezogen, wobei in dem Maschinengehäuse (11) eine achte Sektion (S8) vorgesehen ist, in der mindestens eine Zuführeinrichtung (40) angeordnet ist, wobei die achte Sektion (S8) bezüglich der Transportrichtung der Behälter (B,2) stromaufwärts der fünften Sektion (S5) angeordnet ist, Anordnen von Trennwänden (9) zwischen der achten Sektion (S8) und der fünften Sektion (S5), wobei diese Trennwände (9) der achten Sektion (S8) zusätzlich Durchbrechungen (19) aufweisen, durch welche die Behälter (B,3) von der achten Sektion (S8) zur fünften Sektion (S5) transportiert werden können, Anschließen einer Luftzuführeinrichtung (8) an die achte Sektion (S8) und Zuführen der Luft in die achte Sektion (S8) mittels der Luftzuführeinrichtung (7), derart dass in der achten Sektion (S8) eine Verdrängungsströmung(5) eingerichtet wird, um eine Barriere zur Hallenluft zu erhalten.

14. Vorrichtung zur kontaminationsfreien Behandlung von Behältern, insbesondere Behandlungsmaschine (1), die von einem auf einem Aufstellboden (13) stehenden Maschinengehäuse (11) umgeben ist, wobei die Behandlungsmaschine (11) mindestens eine erste Sektion (S1) umfasst, in der eine Behandlungseinrichtung (4) für Behälter, insbesondere ein Behandlungsrad angeordnet ist, wobei die Vorrichtung umfasst: Trennwände (9), die in der ersten Sektion (S1) und mindestens abschnittweise um die Behandlungseinrichtung (4) herum zur Ausbildung eines von diesen Trennwänden (9) der ersten Sektion (S1) mindestens teilweise umgebenen inneren Abschnittes angeordnet sind, eine Luftzuführeinrichtung (7) für sterile Luft, die an diesen inneren Abschnitt der ersten Sektion (S1) angeschlossen und eingerichtet ist, sterile Luft innerhalb des von den Trennwänden (9) der ersten Sektion (S1) umschlossenen inneren Abschnittes der ersten Sektion (S1) zuzuführen, derart dass ein erster innerer Luftverdrängungsabschnitt (14) ausgebildet wird, der die Behandlungseinrichtung (4) umgibt, wobei die Trennwände (9) der ersten Sektion (S1) mindestens abschnittweise vom Maschinengehäuse (11), insbesondere nach innen beabstandet angeordnet sind, so dass zwischen dem Maschinengehäuse (11) und den Trennwänden (9) äußere Absaugkanäle (12) der ersten Sektion (S1) gebildet werden, welche die Behandlungseinrichtung (4) zu einem überwiegenden Teil umgeben, wobei sich die Trennwände (9) der ersten Sektion (S1) mindestens abschnittweise entlang eines Transportpfades der Behälter (B) um die Behandlungseinrichtung (4) erstrecken und die Behälter (B) in vertikaler Richtung nach oben und unten überragen, ohne mit dem Boden (13), insbesondere Aufstellboden (13), abzuschließen, wobei die Absaugkanäle (12) der ersten Sektion (S1) seitlich und nach oben geschlossen sind und durch das Zuführen steriler Luft in dem ersten inneren Luftverdrängungsabschnitt (14) eine zumindest im Bereich der Behälter (B) vertikale Verdrängungsströmung (5) gebildet wird, wobei eine Luftabführeinrichtung (8) vorgesehen ist, die an die äußeren Absaugkanäle (12) der ersten Sektion (S1) angeschlossen ist und eingerichtet ist, um Luft aus den äußeren Absaugkanälen (12) der ersten Sektion (S1) abzusaugen, so dass eine Saugströmung in den Absaugkanälen (12) der ersten Sektion (S1) gebildet wird und Luft vom ersten inneren Luftverdrängungsabschnitt (14) in radialer Richtung zu den äußeren Absaugkanälen (12) der ersten Sektion (S1) unter den Trennwänden (9) der ersten Sektion (S1) hindurch vorbeifließt.
